# EUROPEAN PATENT APPLICATION

(11) **EP 4 272 752 A1**
(43) Date of publication of application: **08.11.2023**
(21) Application number: 22382424.4
(22) Date of filing: 03.05.2022
(51) Int. Cl.: A61K 38/08, A61P 35/00

(54) **PASIREOTIDE OR A PHARMACEUTICAL ACCEPTABLE SALT OR SOLVATE THEREOF, FOR USE IN PREVENTION OR DELAY OF BREAST CANCER IN A FEMALE MAMMAL**

(71) Applicant: Consejo Superior De Investigaciones Científicas (CSIC), 28006 Madrid (ES)
(72) Inventor: Pérez Losada, Jesús, Madrid (ES); Corchado Cobos, Roberto, Madrid (ES); García Sancha, Natalia, Madrid (ES)
(74) Representative: Manuel Illescas y Asociados, S.L.

(57) **Abstract**

The present invention relates to pasireotide or a pharmaceutically acceptable salt or solvate thereof, for use in prevention or delay of appearance or development of breast cancer or post-pregnancy breast cancer in a female mammal. In addition, the present invention relates to pasireotide long-acting release (pasireotide LAR), preferably pasireotide pamoate, for use in prevention or delay of appearance or development of breast cancer or post-pregnancy breast cancer in a female mammal. Moreover, the present invention also relates to pasireotide long-acting release (pasireotide LAR), preferably pasireotide pamoate, for use in prevention or delay of appearance or development of post-pregnancy breast cancer in a female mammal, wherein said female mammal has weaned their offspring and/or is undergoing postlactational involution.

## Description

### Field of the invention

The present invention relates to the field of oncology, in particular to the field of cancer prevention or delay.

### Background

It is the problem of the present invention to prevent or delay appearance or development of breast cancer in a female mammal, preferably post pregnancy breast cancer. It is also the problem of the present invention to prevent or delay post pregnancy breast cancer in a female mammal who has weaned their offspring and/or is undergoing postlactational involution and/or has a mutation in the *Brca1* gene and/or the *P53* gene and/or the *ErbB2* gene. It is furthermore the problem of the present invention to prevent or delay post pregnancy breast cancer in a woman, preferably a woman who is ≥ 30 years old when having given birth more preferably ≥ 35 years old when having given birth, and/or a woman who was pregnant for more than 34 weeks.

### Brief description of the invention

The present invention relates to pasireotide or a pharmaceutically acceptable salt or solvate thereof, for use in prevention or delay of appearance or development of breast cancer in a female mammal.

In addition, the present invention relates to pasireotide or a pharmaceutically acceptable salt or solvate thereof, for use in prevention or delay of appearance or development of post-pregnancy breast cancer in a female mammal.

Analogously, the present invention relates to a method of prevention or delay of appearance or development of breast cancer or post-pregnancy breast cancer in a female mammal comprising administration thereto of pasireotide or a pharmaceutically acceptable salt or solvate thereof. Similarly, the present invention relates to use of pasireotide or a pharmaceutically acceptable salt or solvate thereof for the manufacture of a medicament for prevention or delay of appearance or development of breast cancer or post-pregnancy breast cancer in a female mammal.

Moreover, the present invention relates to pasireotide long-acting release (pasireotide LAR), preferably pasireotide pamoate, for use in prevention or delay of appearance or development of breast cancer in a female mammal.

Furthermore, the present invention relates to pasireotide long-acting release (pasireotide LAR), preferably pasireotide pamoate, for use in prevention or delay of appearance or development of post-pregnancy breast cancer in a female mammal.

Analogously, the present invention relates to a method of prevention or delay of appearance or development of breast cancer or post-pregnancy breast cancer in a female mammal comprising administration thereto of pasireotide long-acting release (pasireotide LAR), preferably pasireotide pamoate. Similarly, the present invention relates to use of pasireotide long-acting release (pasireotide LAR), preferably pasireotide pamoate, for the manufacture of a medicament for prevention or delay of appearance or development of breast cancer or post-pregnancy breast cancer in a female mammal.

The present invention also relates to pasireotide long-acting release (pasireotide LAR), preferably pasireotide pamoate, for use in prevention or delay of appearance or development of post-pregnancy breast cancer in a female mammal, wherein said female mammal has weaned their offspring and/or is undergoing postlactational involution.

Analogously, the present invention relates to a method of prevention or delay of appearance or development of post-pregnancy breast cancer in a female mammal comprising administration thereto of pasireotide long-acting release (pasireotide LAR), preferably pasireotide pamoate, wherein said female mammal has weaned their offspring and/or is undergoing postlactational involution. Similarly, the present invention relates to use of pasireotide long-acting release (pasireotide LAR), preferably pasireotide pamoate, for the manufacture of a medicament for prevention or delay of appearance or development of post-pregnancy breast cancer in a female mammal, wherein said female mammal has weaned their offspring and/or is undergoing postlactational involution.

### Description of the figures

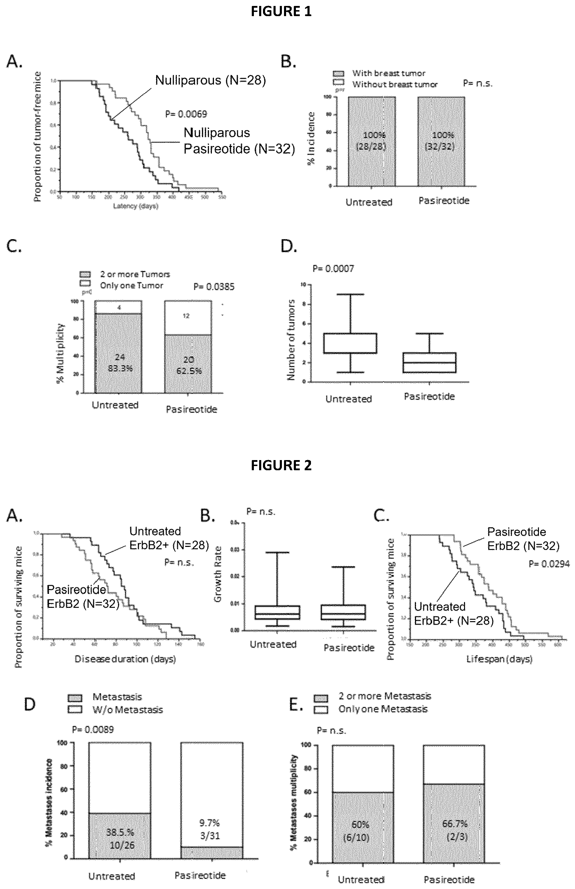

**Figure 1****. Chemoprevention with pasireotide increases resistance to breast cancer in nulliparous mice overexpressing** *ErbB2*/*Neu* **protooncogene. A**) Tumor latency. Kaplan-Meier curves and Log-Rank test. **B**) Tumor incidence. **C**) Multiplicity. B and C, Fisher's test. **D**) Number of tumors. Mann-Whitney U test.
**Figure 2****. Evaluation of tumor evolution in** *ErbB2*/*Neu+* **mice treated with pasireotide. A**) Duration of the disease. **B**) Tumor growth rate. Mann-Whitney U test. **C**) Survival time. **D**) Incidence of metastasis. **E**) Multiplicity of metastases. A and B, Kaplan-Meier curves and Log-Rank test. D and E, Fisher's test.

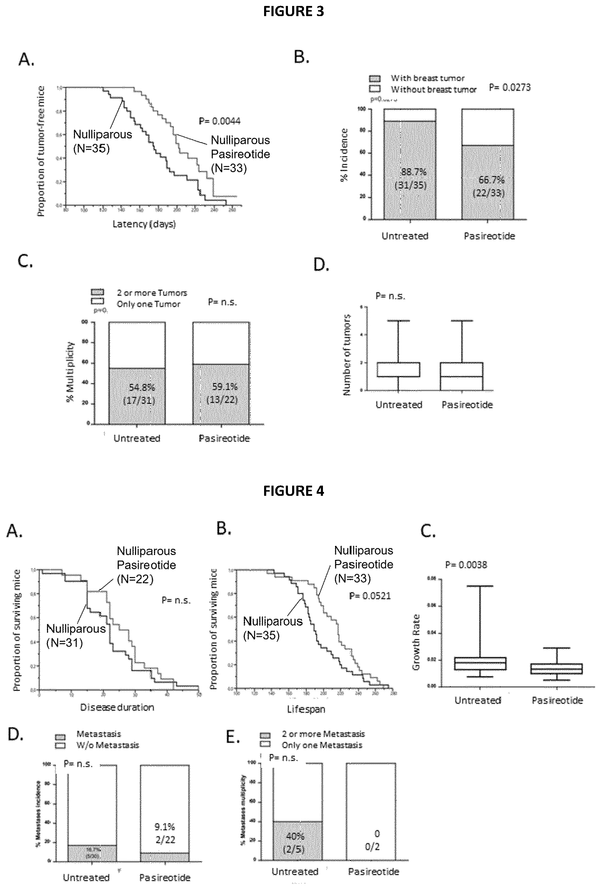

**Figure 3****. Pasireotide treatment increases resistance to mammary cancer in *Brca1*/*P53*-deficient nulliparous mice. A**) Tumor latency. Kaplan-Meier curves and Log-Rank test. **B**) Incidence. **C**) Multiplicity. B and C, Fisher's test. **D**) Number of tumors. Mann-Whitney U test.
**Figure 4****. Evaluation of tumor evolution in nulliparous mice deficient in *Brca1*/*P53* treated with pasireotide. A**) Duration of the disease. **B**) Survival time. A, C, Kaplan-Meier curves and Log-Rank test. **C**) Tumor growth rate. Mann-Whitney U test. **D**) Incidence of metastasis. **E**) Multiplicity of metastases. D and E, Fisher's test.

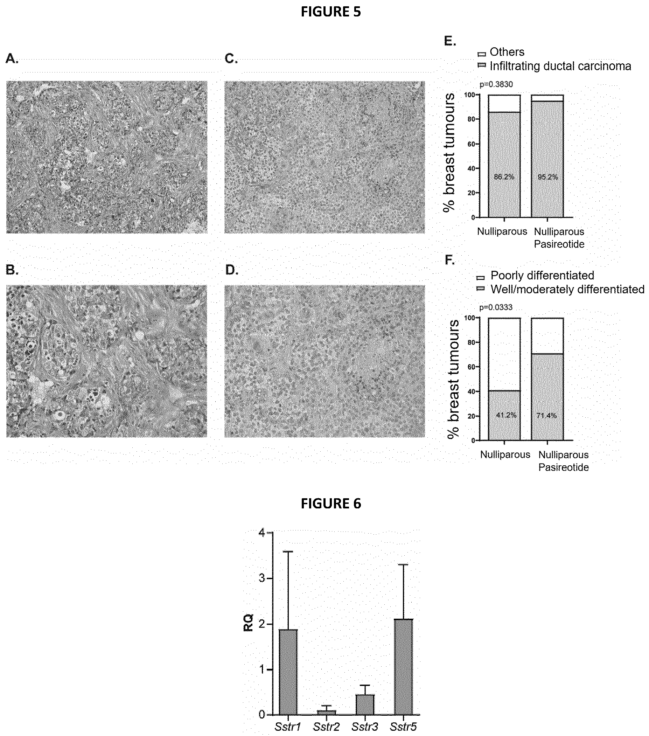

**Figure 5****. Histopathological evaluation of tumors developed in nulliparous, untreated and pasireotide-treated Brca1/P53-deficient mice.** (A) Undifferentiated tumor with sclerosing reaction (20X). (B) Detail of the fibrous bundles of the tumor (40X). (C) Infiltrating ductal carcinoma, moderately differentiated, with a nodular pattern (20X). (D) Detail of focal areas of comedocarcinoma (40X). (E) Comparison of histopathologic tumor type between pasireotide-treated and untreated nulliparous. Fisher's exact test. (F) Comparison of the degree of tumor differentiation between the same groups of mice. Fisher's exact test.
**Figure 6****. Expression of somatostatin receptors in nulliparous ErbB2/Neu mice.** Calculation of the RQ of the four Sstr evaluated. Mouse brain was used as a positive expression control.

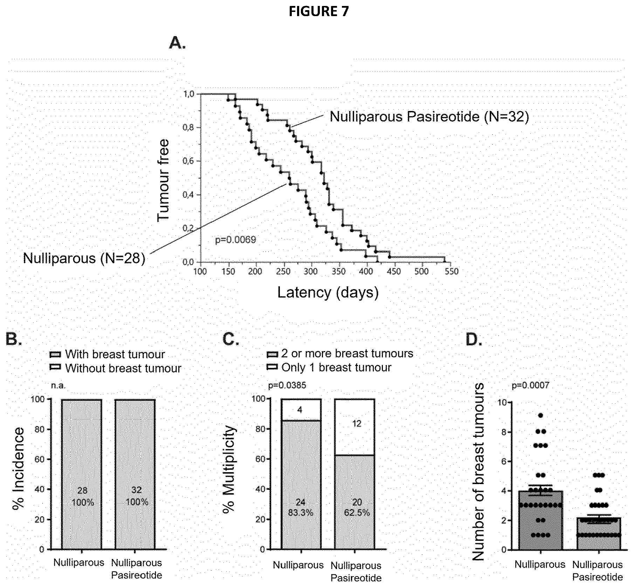

**Figure 7****. Assessment of tumor susceptibility in mice with overexpression of ErbB2/Neu, in nulliparous treated and not treated with pasireotide.** (A) Comparison of tumor latency. Kaplan-Meier estimator and Log-Rank test. (B) Comparison of tumor incidence. Fisher's exact test. (C) Comparison of tumor multiplicity. Fisher's exact test. (D) Comparison of the total number of tumors. Mann-Whitney U test.

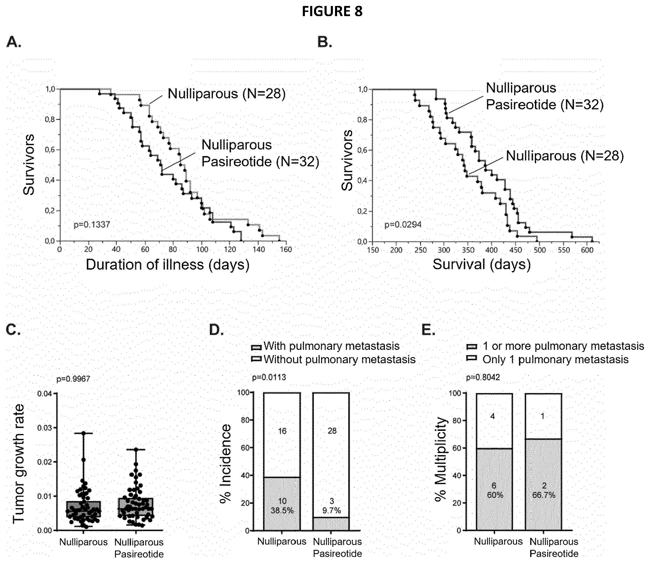

**Figure 8****. Assessment of tumor progression in ErbB2/Neu nulliparous, untreated and pasireotide-treated mice with overexpression.** (A) Comparison of duration of illness. Kaplan-Meier estimator and Log-Rank test. (B) Comparison of survival. Those animals that died from mammary tumors are shown with a black dot. Kaplan-Meier estimator and Log-Rank test. (C) Comparison of tumor growth rate. Mann-Whitney U test. (D) Comparison of the incidence of metastases. Fisher's exact test. (E) Comparison of the multiplicity of metastases. Fisher's exact test.

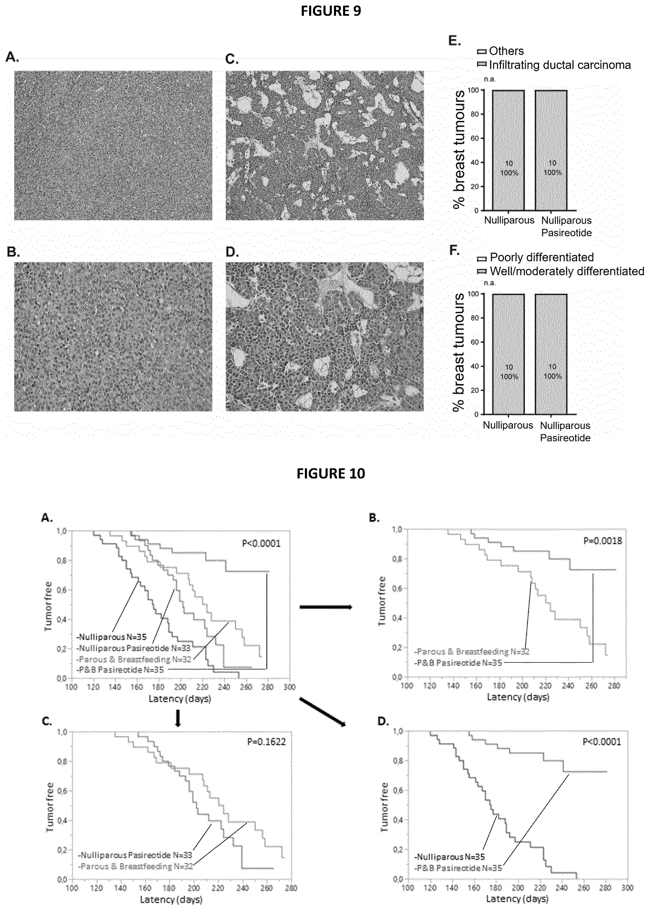

**Figure 9****. Histopathological evaluation of tumors developed in ErbB2/Neu overexpressing mice, in nulliparous treated and untreated with pasireotide.** (A) Infiltrating ductal adenocarcinoma with a solid pattern (10X). (B) Infiltrating ductal adenocarcinoma with a solid pattern (20X). (C) Infiltrating ductal adenocarcinoma with cribriform pattern (10x). (D) Infiltrating ductal adenocarcinoma with cribriform pattern (20x).
**Figure 10****. Pasireotide potentiates the protective effect of pregnancy in the Brca1/P53 deficient mouse model in terms of tumor latency. A-D**) Comparison of tumor latency in A) all groups; B) parous mice treated with pasireotide versus parous untreated mice; C) nulliparous mice treated with pasireotide versus parous mice; D) parous mice treated with pasireotide versus nulliparous untreated mice. A-C, Kaplan-Meier Curves, and log. Rank test.

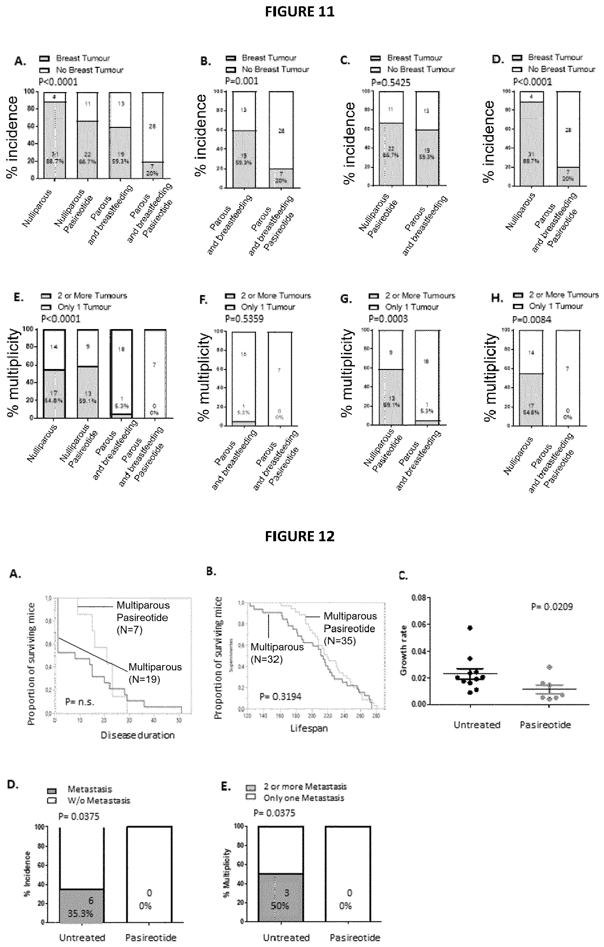

**Figure 11****. Pasireotide potentiates the protective effect of pregnancy in the *Brca1*/*P53* deficient mouse model in terms of tumor incidence and multiplicity. A-D**) Comparison of tumor incidence in **A**) all groups; **B**) parous mice treated with pasireotide versus parous untreated mice; **C**) nulliparous mice treated with pasireotide versus parous mice; **D**) parous mice treated with pasireotide versus nulliparous untreated mice. **E-H**) Comparison of tumor multiplicity in **E**) all groups; **F**) parous mice treated with pasireotide versus parous untreated mice; **G**) nulliparous mice treated with pasireotide versus parous mice; **H**) parous mice treated with pasireotide versus nulliparous untreated mice. A-H. Fisher's test.
**Figure 12****. Evaluation of tumor evolution in multiparous mice deficient in *Brca1*/*P53* treated with pasireotide. A**) Duration of the disease. **B**) Survival time. A and B, Kaplan-Meier curves and Log-Rank test. **C** Tumor growth rate. Mann-Whitney U test. **D**) Incidence of metastasis. **E**) Multiplicity of metastases. D, E, Chi-square test.

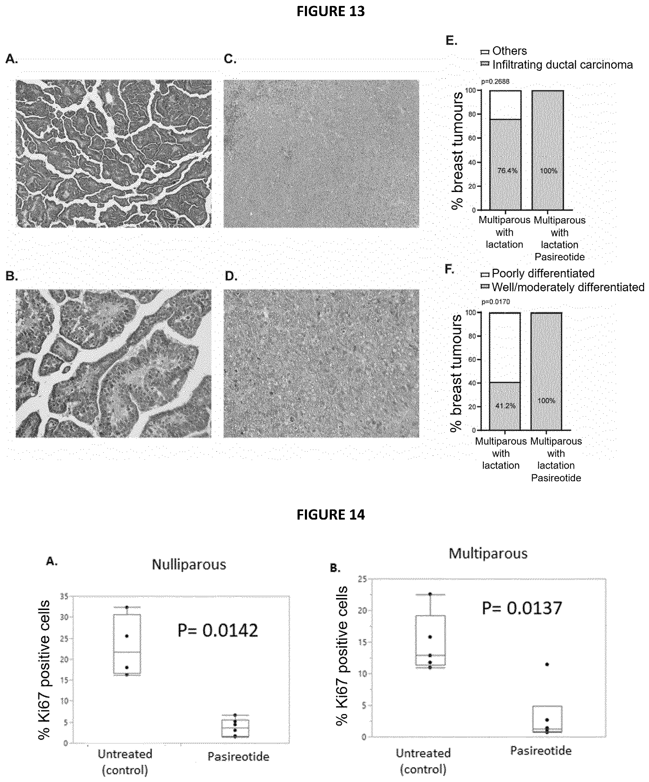

**Figure 13****. Histopathological evaluation of tumors developed in multiparous Brca1/P53 knockout mice, treated and untreated with pasireotide.** (A) Well-differentiated tumor (20X). (B) Well-differentiated tumor (40X). (C) Poorly differentiated tumor (20X). (D) Poorly differentiated tumor (40X). (E) Comparison of tumor histopathology between pasireotide-treated and untreated multiparous. Fisher's exact test. (F) Comparison of the degree of tumor differentiation between untreated multiparous mice and multiparous mice treated with pasireotide. Fisher's exact test.
**Figure 14****. Evaluation of proliferation (Ki67) after treatment with pasireotide in mice at five months of age (equivalent to 60 days post-weaning in multiparous mice). A,** B) Comparison between treated and untreated female mice **A**) nulliparous; **B**) multiparous. 5-6 mice per group and five breast fields per mouse were compared (Mann-Whitney U test).

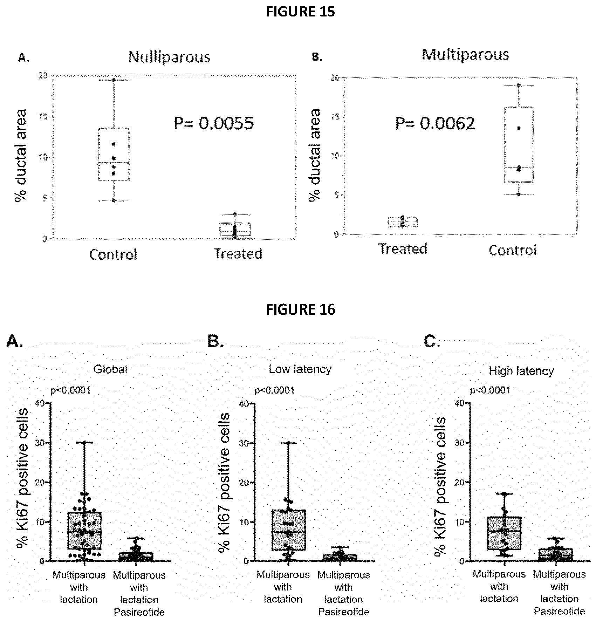

**Figure 15****. Evaluation of the ductal wall area after treatment with pasireotide in nulliparous and multiparous mice at five months. A, B**) Comparison between treated and untreated female mice **A**) nulliparous; **B**) multiparous. Mann-Whitney U test.
**Figure 16****. Comparison of the degree of ductal epithelial proliferation between Brca1/P53-deficient multiparous, pasireotide-treated and non-treated mice.** (A) Comparison of epithelial proliferation between multiparous treated and untreated with pasireotide, including all mice. (B) Comparison of epithelial proliferation between multiparous mice with low tumor latency treated and untreated with pasireotide. (C) Comparison of proliferation between multiparous with high tumor latency, treated and not treated with pasireotide. Mann-Whitney U test.

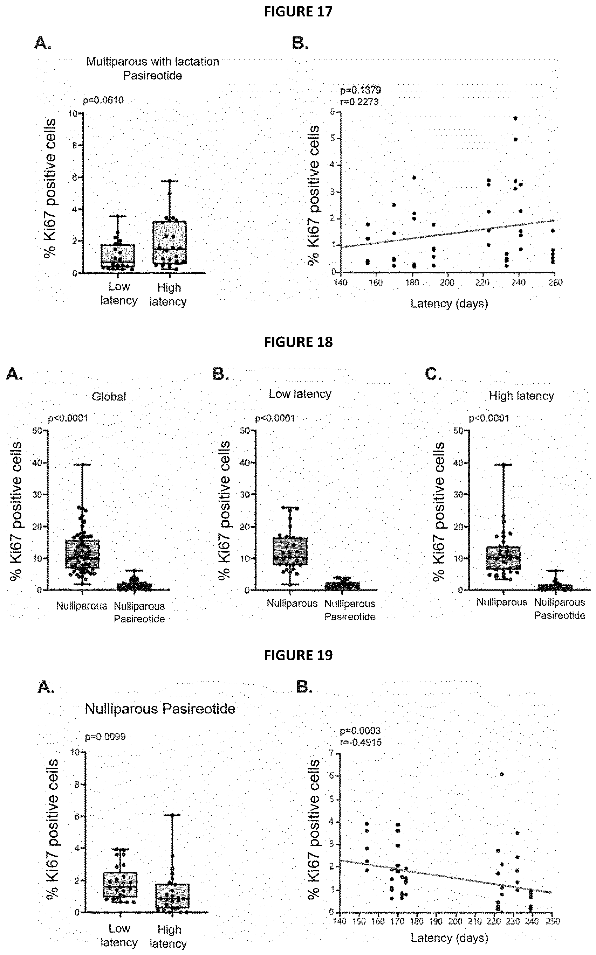

**Figure 17****. Evaluation of the relationship between the degree of epithelial proliferation and tumor latency in multiparous mice, treated with pasireotide, deficient in Brca1/P53.** (A) Comparison of mammary epithelial proliferation in pasireotide-treated multiparous mice, between animals with high and low tumor latency. Mann-Whitney U test. (B) Correlation between the degree of epithelial proliferation and tumor latency in multiparous mice treated with pasireotide. Spearman test.
**Figure 18****. Comparison of the degree of epithelial proliferation in the breast between Brca1/P53-deficient pasireotide-treated and untreated nulliparous mice.** (A) Comparison of epithelial proliferation between treated and untreated gilts with pasireotide, considering all animals. (B) Comparison of epithelial proliferation among nulliparous with low tumor latency. (C) Comparison between nulliparous with high tumor latency. Mann-Whitney U test.
**Figure 19****. Evaluation of the relationship between the degree of epithelial proliferation and intragroup tumor latency, in nulliparous mice treated with pasireotide, deficient in Brca1/P53.** (A) Comparison of epithelial proliferation between pasireotide-treated nulliparous mice of high and low tumor latency. Mann-Whitney U test. (B) Correlation between the degree of epithelial proliferation and tumor latency in nulliparous treated with pasireotide. Spearman test.

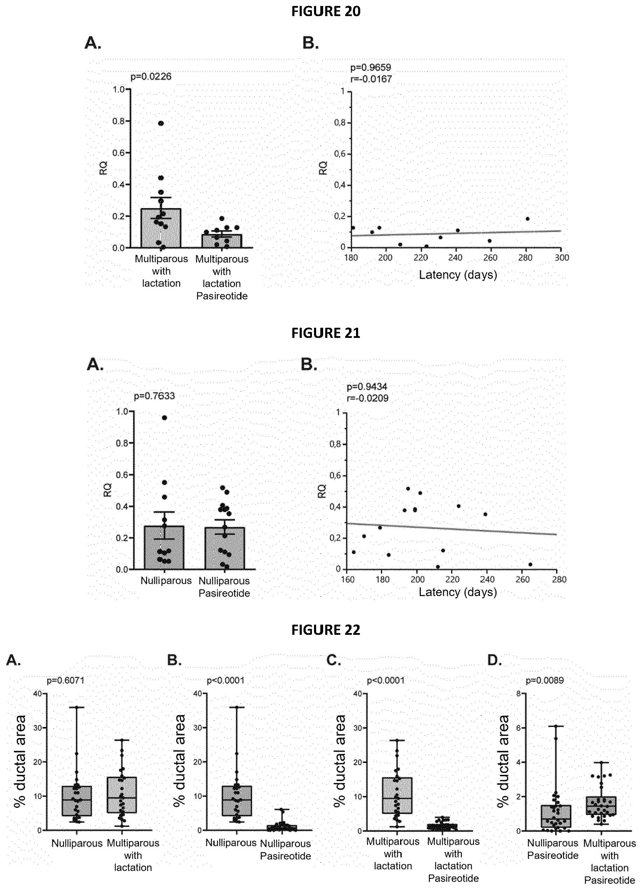

**Figure 20****. Quantification of the recombined P53 allele in the non-tumorous breast of pasireotide-treated, Brca1/P53-deficient multiparous mice.** (A) Comparison of the relative amount (RQ) of recombined allele of P53 between treated and untreated lactating multiparous with pasireotide. Mann-Whitney U. (B) Correlation between the RQ of the P53 recombinant allele and tumor latency in pasireotide-treated multiparous mice. Spearman test. As a positive control, breast tumor was used.
**Figure 21****. Quantification of the recombined allele of P53 in the non-tumorous breast of nulliparous mice, deficient in Brca1/P53, treated with pasireotide.** (A) Comparison of the relative amount (RQ) of recombined allele of P53 between pasireotide treated and untreated gilts. Mann-Whitney U. (B) Correlation between the RQ of the P53 recombinant allele and tumor latency in nulliparous mice treated with pasireotide. Spearman test. As a positive control, breast tumor was used.
**Figure 22****. Comparison of the ductal area in the different groups of the cross-sectional cohort. (A) Nulliparous versus multiparous, both untreated.** (B) Untreated nulliparous versus pasireotide-treated nulliparous. (C) Untreated multiparous versus multiparous treated with pasireotide. (D) Nulliparous versus multiparous, both groups treated with pasireotide. Mann-Whitney U test.

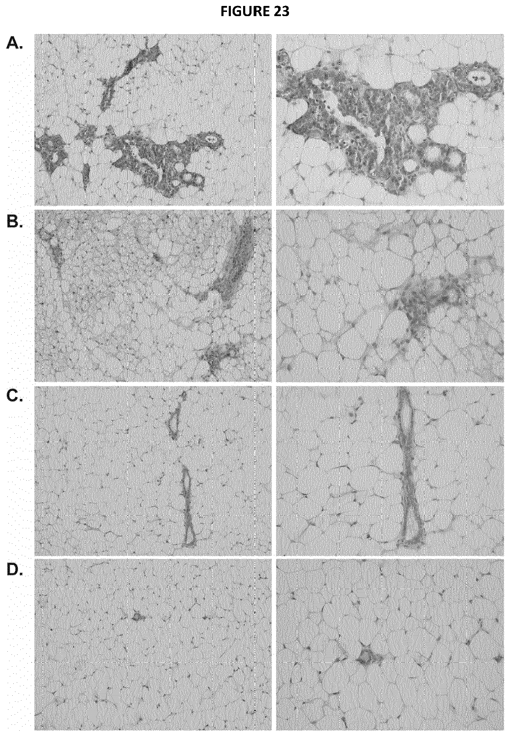

**Figure 23****. Photos of normal breasts from the different groups of the cross-sectional cohort.** (A) Nulliparous without treatment. Left, 20X. Right, 40X. (B) Multiparous without treatment. Left, 20X. Right, 40X. (C) Nulliparous with treatment. Left, 20X. Right, 40X. (D) Multiparous with treatment. Left, 20X. Right, 40X.

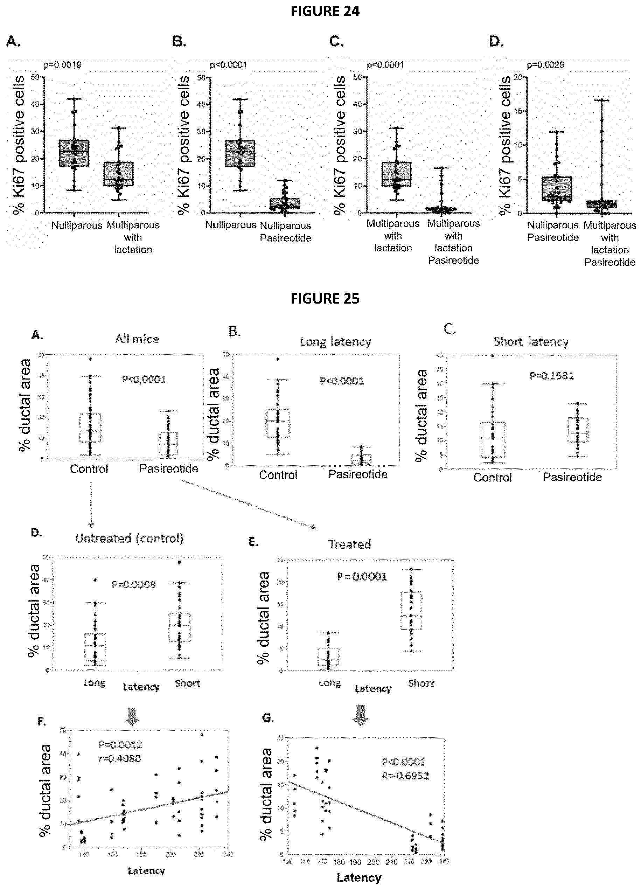

**Figure 24****. Comparison of ductal epithelial proliferation in the different groups of the cross-sectional cohort.** (A) Nulliparous versus multiparous. (B) Untreated nulliparous versus pasireotide-treated nulliparous. (C) Untreated multiparous versus multiparous treated with pasireotide. (D) Nulliparous treated with pasireotide versus multiparous treated with pasireotide. Mann-Whitney U test.
**Figure 25****. Quantification of the ductal component in the longitudinal cohort in nulliparous *Brca1*/*P53* deficient mice** (This figure is constructed considering fields). **A-C**) Comparison of the ductal area between mice treated and untreated with pasireotide. **A**) In all mice. **B**) In mice with long latency tumors. **C**) In cases of low latency. **D, E**) Comparison of the ductal area between mice that developed breast tumors with high and low latency; **D**) in control mice, not treated; **E**) in mice treated with pasireotide. **F, G**) Correlation between tumor latency and ductal area, **F**) in untreated mice; **G**) in treated mice. A-E, Student's t-test; F, G, Pearson's correlation test.

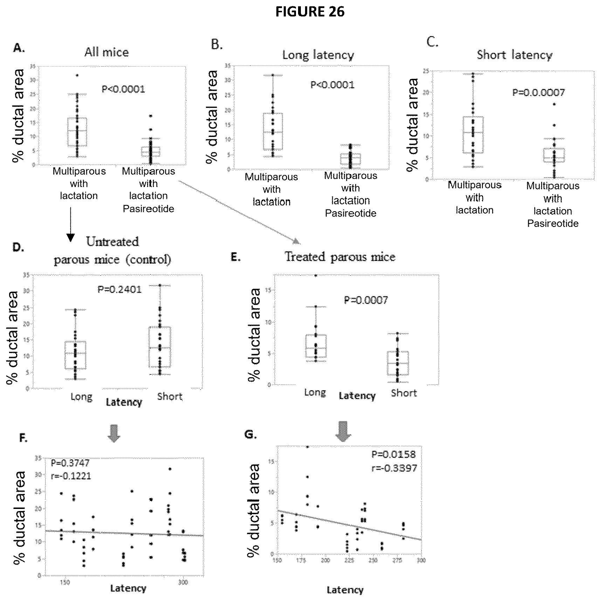

**Figure 26****. Quantification of the ductal component in the non-tumorous mammary glands from multiparous** Brca1/P53-deficient **mice from the longitudinal cohort treated with pasireotide.** (This figure is constructed considering fields). **A-C**) Comparison of the ductal area between multiparous mice treated and untreated with pasireotide. **A**) In all mice. **B**) In mice with long latency tumors. **C**) In cases of low latency. **D, E**) Comparison of the ductal area between mice that developed breast tumors with high and low latency, **D**) in control mice, not treated; **E**) in mice treated with pasireotide. **F, G**) Correlation between tumor latency (days) and ductal area, **F**) in untreated mice; G) in treated mice. A-E, Student's t-test; F, G, Pearson's correlation test.

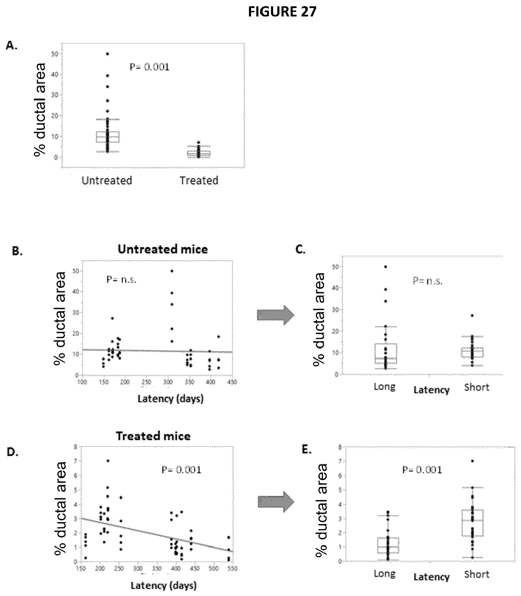

**Figure 27****. Quantification of the ductal component area in the non-tumorous mammary glands of treated and untreated (control)** *ErbB2*/*Neu+* **nulliparous mice (the longitudinal cohort). A**) Comparison of the ductal area between treated and untreated *ErbB2*/*Neu+* nulliparous mice. **B**) Absence of correlation between ductal area and tumor latency in untreated mice (Pearson). **C**) Comparison of the ductal area in non-tumoral mammary glands in untreated mice with short and long breast cancer latency. **D**) The same as B, but in treated mice. **E**) The same as C but in treated mice.

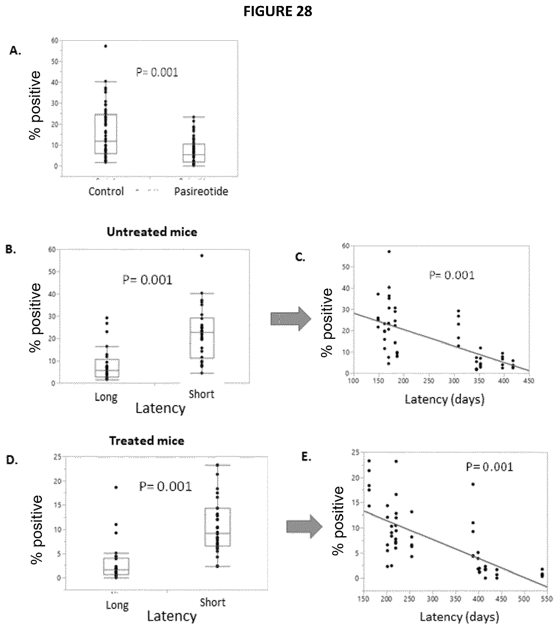

**Figure 28****. Quantification of the ductal component proliferation in the non-tumorous mammary glands of treated and untreated (control)** *ErbB2*/*Neu+* **nulliparous mice (the longitudinal cohort). A**) Comparison of the proliferation between treated and untreated *ErbB2*/*Neu+* nulliparous mice. **B**) Correlation between proliferation and tumor latency in untreated mice (Pearson). **C**) Comparison of the ductal area in non-tumoral mammary glands in untreated mice with short and long breast cancer latency. **D**) The same as B, but in treated mice. **E**) The same as C but in treated mice.

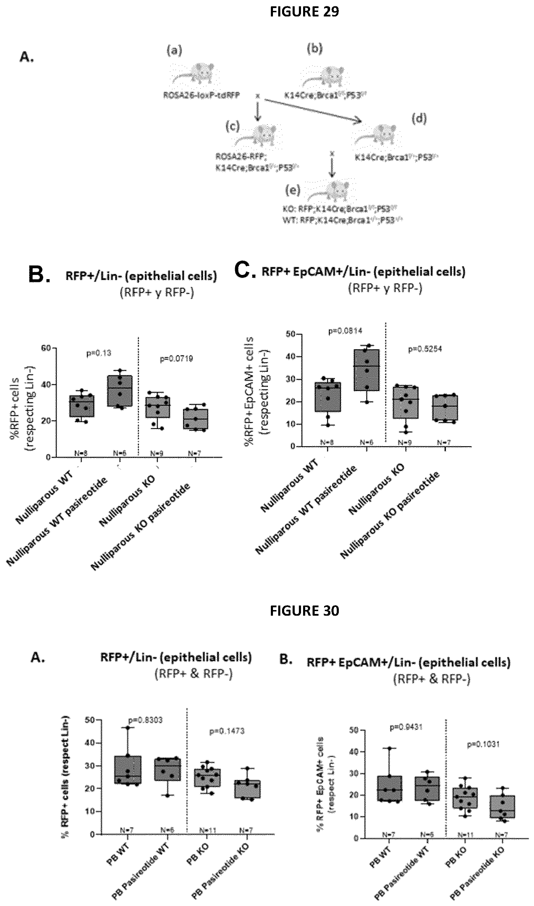

**Figure 29****. Recombinant cell quantification by flow cytometry in nulliparous *Brca1*/*P53*-deficient mice. A)** Scheme of crossing to obtain mice in which the recombinant cells emit red fluorescence. **(a)** The "fluorescent" (RFP) mouse expresses the discosome Red Fluorescent Protein (dsRed) in a tandem dimer (dtRFP) under the ROSA26 promoter. A stop codon precedes the RFP cDNA. These mice were crossed with **(b)** the *Brca1*/*P53* deficient mice, which are also transgenic for Cre recombinase under the cytokeratin 14 promoter (K14-Cre), which eliminates *Brca1* and *P53* in a percentage of the cells of the mammary gland; **(c)** in the F1 generation, transgenic mice for K14-Cre were obtained and remove the stop codon and cause RFP to be expressed. Cre-recombinase simultaneously removes the *Brca1* and *P53* alleles, which remain heterozygous. **(d)** Among the F1 offspring, there are also mice heterozygous for the loss of *Brca1* and *P53.* **(e)** Mice from "c" and "d" were crossed to obtain rodents that lose both alleles of *Brca1* and *P53.* RFP+ mice without loss of the *Brca1*/*P53* alleles were also generated (WT or wild type). **B**) Comparison of the proportion of recombinant cells (RFP+) compared to epithelial cells (Lin-). **C**) Comparison of the proportion of recombinant cells (EpCAM+RFP+) compared to epithelial cells (Lin-). Mann-Whitney U test.
**Figure 30****. Recombinant cell quantification by flow cytometry in multiparous *Brca1*/*P53-*deficient mice. A**) Comparison of the proportion of recombinant cells (RFP+) compared to epithelial cells (Lin-). **B**) Comparison of the proportion of recombinant cells (EpCAM+RFP+) compared to epithelial cells (Lin negative). Mann-Whitney U test. PB, multiparous, and breastfeeding.

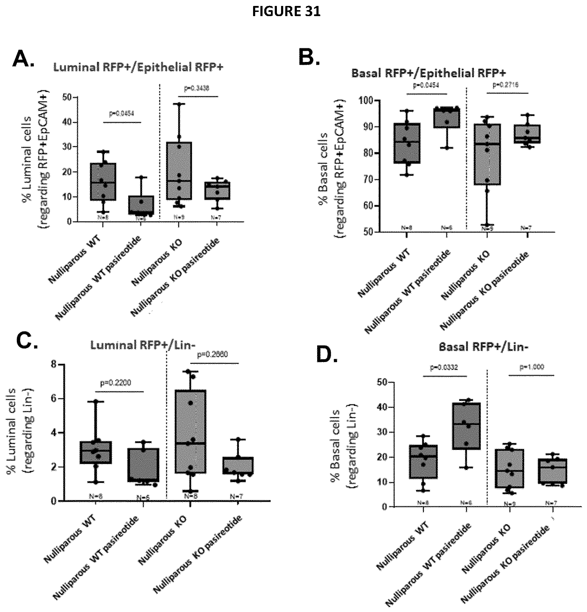

**Figure 31****. Evaluation of the proportion of the luminal and basal components in nulliparous *Brca1*/*P53* deficient mice. A, B**) Luminal and basal cells within the recombinant epithelial cells (RFP+ EpCAM+), **C, D**) and within the Lin- cells. Mann-Whitney U test.

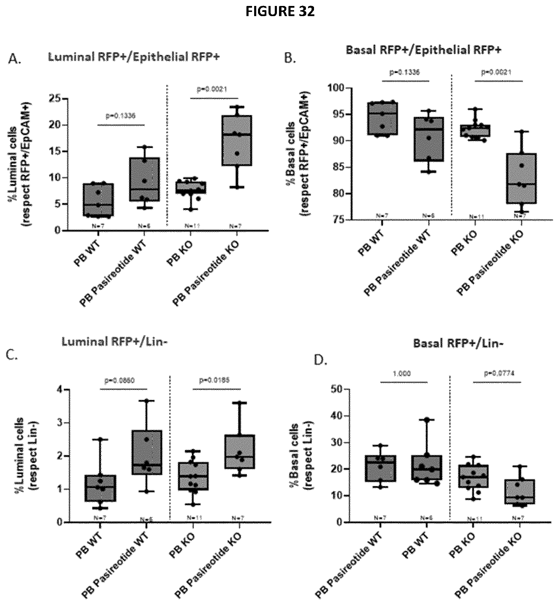

**Figure 32****. Evaluation of the luminal and basal components proportion in multiparous *Brca1*/*P53* deficient mice. A, B**) Luminal and basal cells within the recombinant epithelial cells (RFP+ EpCAM+), **C, D**) and within the Lin- cells. Mann-Whitney U test.

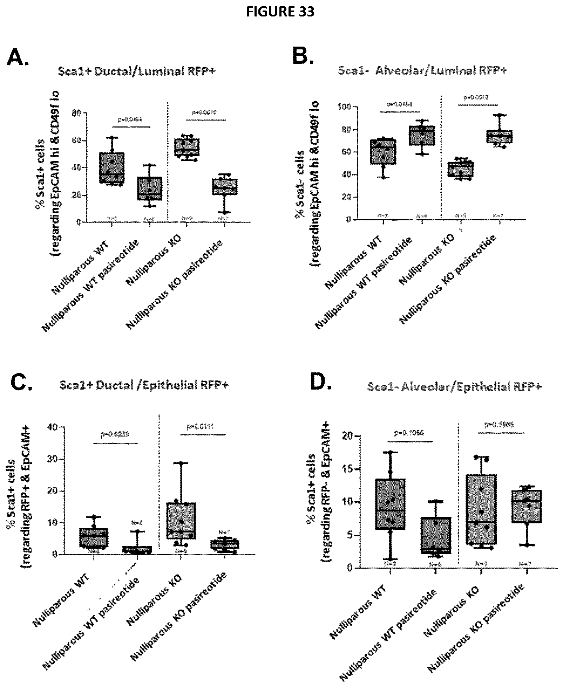

**Figure 33****. Evaluation of the proportion of luminal subpopulations: ductal and alveolar in nulliparous *Brca1*/*P53* deficient mice. A, B)** Comparison between groups of B) ductal cells and C) alveolar cells concerning the total number of recombinant luminal cells (EpCAM hi/CD49f lo/RFP+). **C, D)** Comparison of the proportion of ductal cells **C)** ductal and **D)** alveolar concerning the total number of recombinant epithelial cells (RFP+ EpCAM+). Mann-Whitney U test.

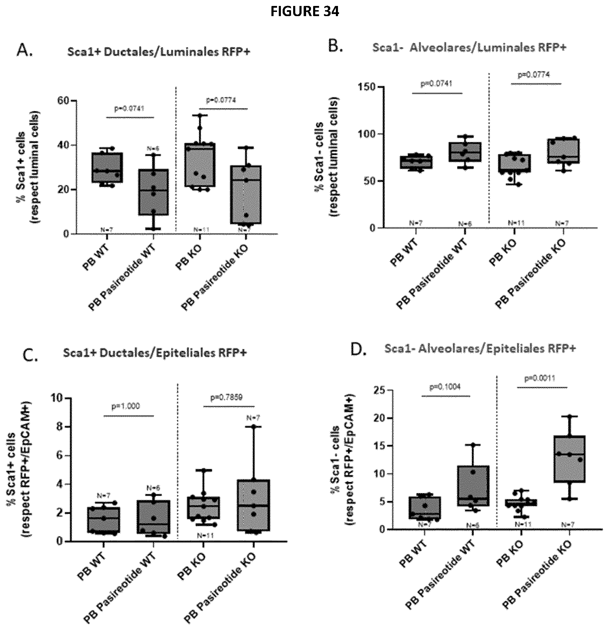

**Figure 34****. Evaluation of the proportion of luminal subpopulations: ductal and alveolar in multiparous *Brca1*/*P53* deficient mice. A, B)** Comparison between groups of B) ductal cells and C) alveolar cells for the total number of recombinant luminal cells (EpCAM hi/CD49f Io/RFP+). **C, D)** Comparison of the proportion of ductal cells **C)** ductal and **D)** alveolar to the total number of recombinant epithelial cells (RFP+ EpCAM+). Mann-Whitney U test. PB, parous (multiparous), and breastfeeding mice.

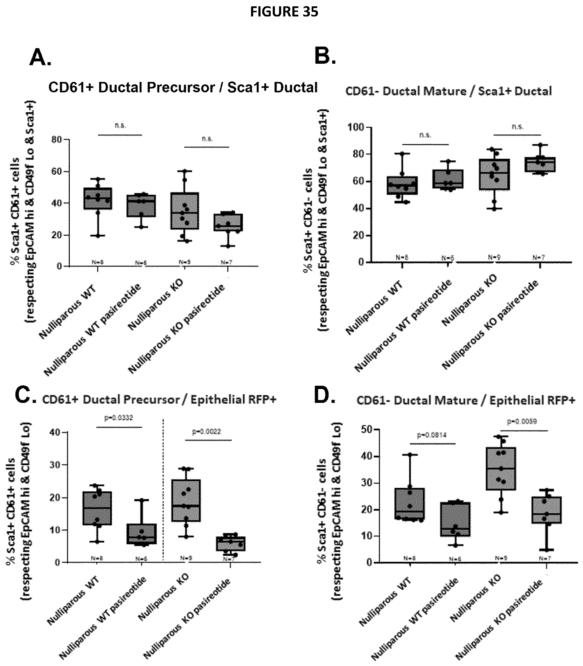

**Figure 35****. Evaluation of the proportion of ductal precursors and mature subpopulations in nulliparous *Brca1*/*P53* deficient mice. A, B)** Comparison between groups of **A**) ductal precursors and **B**) mature ductal cells for the total number of recombinant ductal cells (EpCAM hi/CD49f Io/Sca1+/RFP+). **C, D)** Comparison of the proportion of ductal cells **D**) ductal and **E**) alveolar for the total of the recombinant epithelial cells (RFP+ EpCAM+). Mann-Whitney U test.

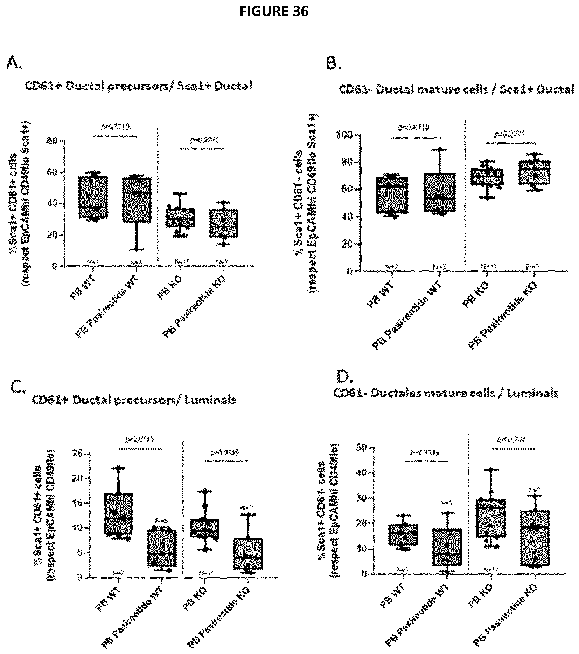

**Figure 36****. Evaluation of the proportion of ductal precursors and mature subpopulations in multiparous *Brca1*/*P53* deficient mice. A, B)** Comparison between groups of B) ductal precursors and C) mature ductal cells for the total number of recombinant ductal cells (EpCAM hi/CD49f Io/Sca1+/RFP+). **D, E)** Comparison of the proportion of ductal cells D) ductal and E) alveolar for the total of the recombinant epithelial cells (RFP+ EpCAM+). Mann-Whitney U test.

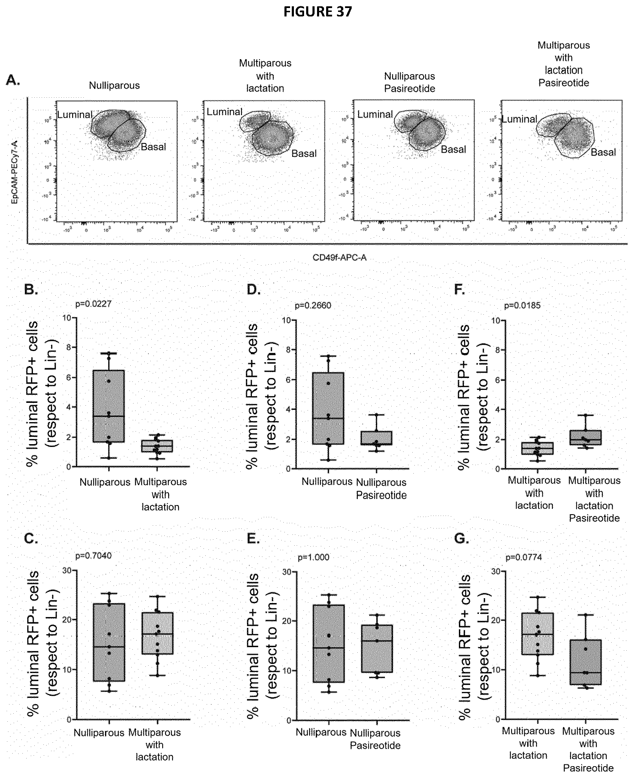

**Figure 37****. Evaluation of the effect of pregnancy and pasireotide on recombinant luminal and basal cell subpopulations. In all groups of the cross-sectional cohort, the proportion of recombinant luminal and basal cells (RFP+) with respect to the total number of Lin negative cells was evaluated.** (A) Flow cytometry dot plot of the luminal and basal subpopulations in the different groups of mice. (B) Comparison of the proportion of RFP+ luminal cells between nulliparous and multiparous. (C) Comparison of RFP+ basal cells between nulliparous and multiparous. (D) Comparison of RFP+ luminal cells between untreated and pasireotide-treated gilts. (E) Comparison of RFP+ basal cells between untreated and pasireotide-treated gilts. (F) Comparison of RFP+ luminal cells between multiparous treated and untreated with pasireotide. (G) Comparison of RFP+ basal cells between multiparous and multiparous treated with pasireotide. Mann-Whitney U test.

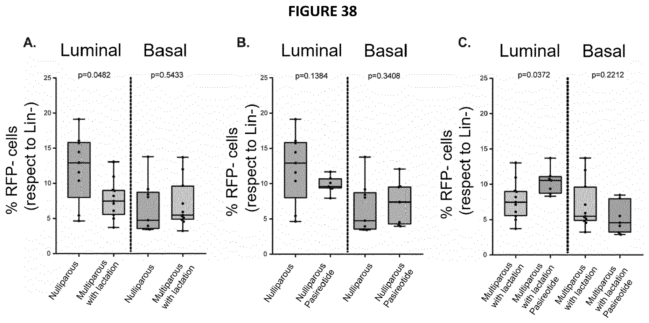

**Figure 38****. Evaluation of the effect of pregnancy and pasireotide on the luminal and basal subpopulations in non-recombinant cells.** In all groups of the cross-sectional cohort, the proportion of non-recombinant luminal and basal (RFP-) cells with respect to the total Lin negative cells was evaluated (A) Comparison of luminal and basal RFP- cells between nulliparous and multiparous. (B) Comparison of luminal and basal RFP- cells between untreated and pasireotide-treated gilts. (C) Comparison of luminal and basal RFP- cells between multiparous treated and untreated with pasireotide. Mann-Whitney U test.

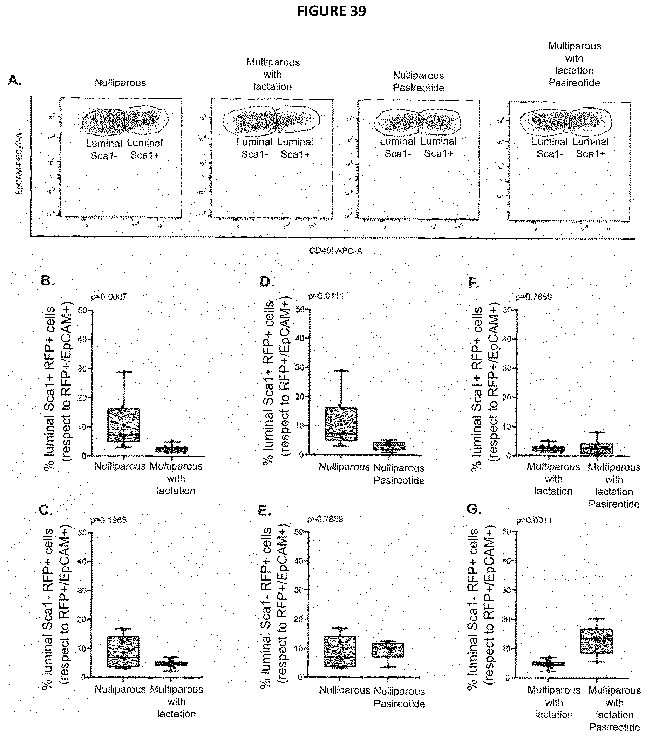

**Figure 39****. Evaluation of the effect of pregnancy and pasireotide on recombinant luminal, ductal (Sca1+), and alveolar (Sca1-) subpopulations.** In all groups of the cross-sectional cohort, the proportion of recombinant luminal ductal and alveolar cells (RFP+) with respect to the total number of recombinant EpCAM+ cells was evaluated. (A) Dot plot of the ductal and alveolar populations in the different groups of mice of the cross-sectional cohort. (B) Comparison of the proportion of Sca1+ ductal cells between nulliparous and multiparous; (C) Comparison of Sca1- alveolar cells between nulliparous and multiparous; (D) Comparison of ductal cells between nulliparous treated and untreated with pasireotide; (E) Comparison of alveolar Sca1- cells between treated and untreated gilts; (F) Comparison of Sca1+ ductal cells between treated and untreated multiparous; and (G) Comparison of Sca1- alveolar cells between treated and untreated multiparous Mann Whitney U Test.

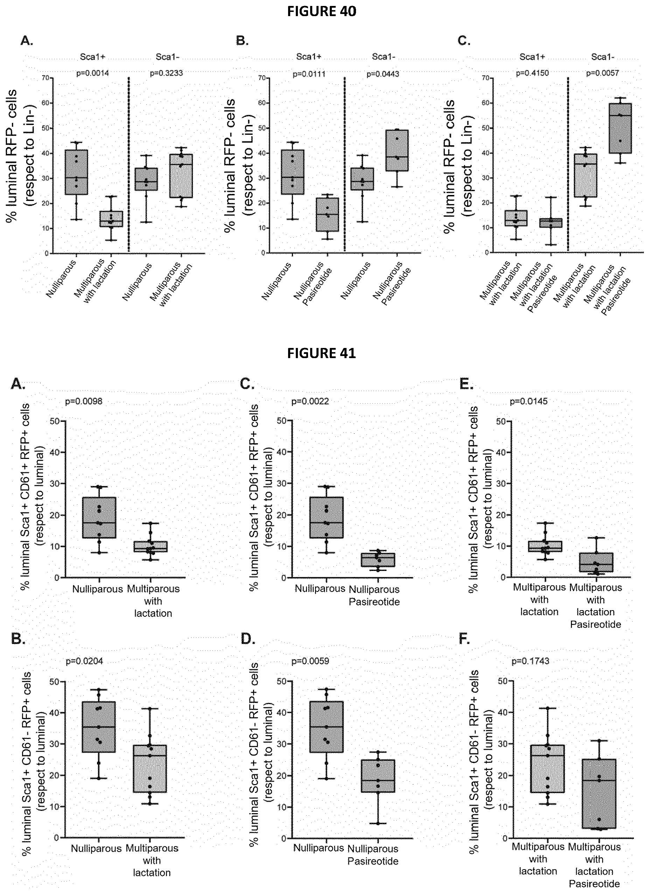

**Figure 40****. Evaluation of the effect of pregnancy and pasireotide on the non-recombinant luminal ductal (Sca1+) and alveolar (Sca1-) population.** In all groups of the cross-sectional cohort, the proportion of non-recombinant ductal and alveolar luminal cells (RFP-) with respect to the total number of non-recombinant EpCAM+ cells was evaluated. (A) Comparison of RFP- ductal and alveolar cells between nulliparous and multiparous. Left, Sca1+ and, right, Sca1-. (B) Comparison of ductal and alveolar RFP- cells between nulliparous treated and untreated with pasireotide. Left, Sca1+ and, right, Sca1-. (C) Comparison of ductal and alveolar RFP- cells between multiparous and multiparous treated with pasireotide. Left, Sca1+ and, right, Sca1-. Mann-Whitney U test.
**Figure 41****. Evaluation of the effect of pregnancy and pasireotide on the population of recombinant ductal progenitors (CD61+) and mature ductal cells (CD61-).** In all groups of the cross-sectional cohort, the proportion of recombinant progenitor and mature luminal ductal cells was evaluated with respect to the total number of RFP+ luminal cells. (A) Comparison of CD61+ progenitors between nulliparous and multiparous mice; (B) CD61-mature cells between nulliparous and multiparous; (C) of CD61+ progenitors between pasireotide treated and untreated gilts; (D) CD61-mature cells between treated and untreated gilts; (E) of CD61+ progenitors between pasireotide-treated and untreated multiparous; and (F) of mature CD61- cells between multiparous and multiparous treated with pasireotide. Mann-Whitney U test.

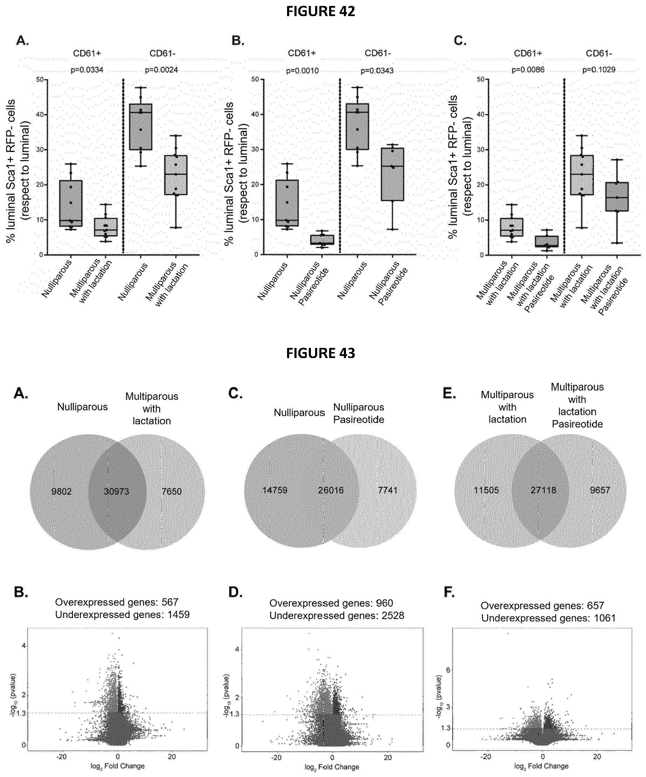

**Figure 42****. Evaluation of the effect of pregnancy and pasireotide on the population of** non-**recombinant ductal progenitors (CD61+) and mature ductal cells (CD61-).** In all groups of the cross-sectional cohort, the proportion of progenitor and mature luminal ductal cells, non-recombinant, with respect to the total number of luminal RFP- cells was evaluated. (A) Comparison of RFP-mature ductal and ductal progenitor cells between nulliparous and multiparous. Left, CD61+ and right, CD61-(B) Comparison of RFP-mature ductal and ductal progenitor cells between nulliparous and pasireotide-treated nulliparous. Left, CD61+ and right, CD61-. (C) Comparison of RFP-mature ductal and ductal progenitor cells between multiparous and pasireotide-treated multiparous. Left, CD61+ and right, CD61-. Mann-Whitney U test.
**Figure 43****. Evaluation of differentially expressed genes between the different study groups of the cross-sectional cohort.** (A) Venn diagram showing specifically and shared genes expressed in nulliparous and multiparous mice. (B) Volcano plot analysis with the number of differential genes overexpressed and underexpressed in multiparous compared to nulliparous. (C) Venn diagram showing genes specifically expressed and shared between pasireotide-treated and untreated gilts. (D) Volcano plot analysis with differential overexpressed and underexpressed genes in pasireotide-treated gilts relative to untreated gilts. (E) Venn diagram showing genes specifically expressed and shared between multiparous treated and untreated with pasireotide. (F) Volcano plot analysis with differential overexpressed and underexpressed genes in pasireotide-treated multiparous versus untreated multiparous.

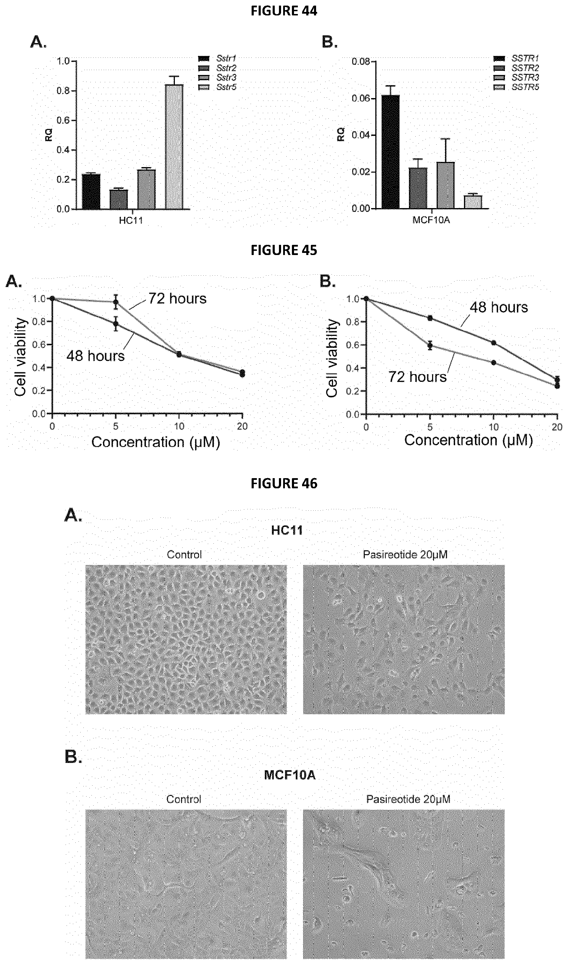

**Figure 44****. Quantification of the expression level of somatostatin receptors in non-tumor cell lines.** (A) Calculation of the RQ of the Sstr in HC11 cells. Mouse brain RNA was used as a positive control. (B) Calculation of the RQ of SSTR receptors in MCF10A cells. Human cutaneous epidermoid tumor RNA was used as positive control.
**Figure 45****. Evaluation of cell viability in the presence of pasireotide at 5 µM, 10 µM and 20 µM, in non-tumor lines.** (A) MTT assay in HC11 cells at 48 and 72 hours. (B) MTT in MCF10A cells at 48 and 72 hours. The assay was performed in triplicate.
**Figure 46****. Effect of pasireotide on cell growth in non-tumor breast cells. Images obtained under a phase-contrast microscope at 10X magnification, of untreated cells and cells treated at a 20 µM concentration of pasireotide for 48 hours.** (A) Left panel shows untreated HC11 cells; and on the right treated HC11 cells. (B) Left panel shows untreated MCF10A cells; and on the right, treated MCF10A cells.

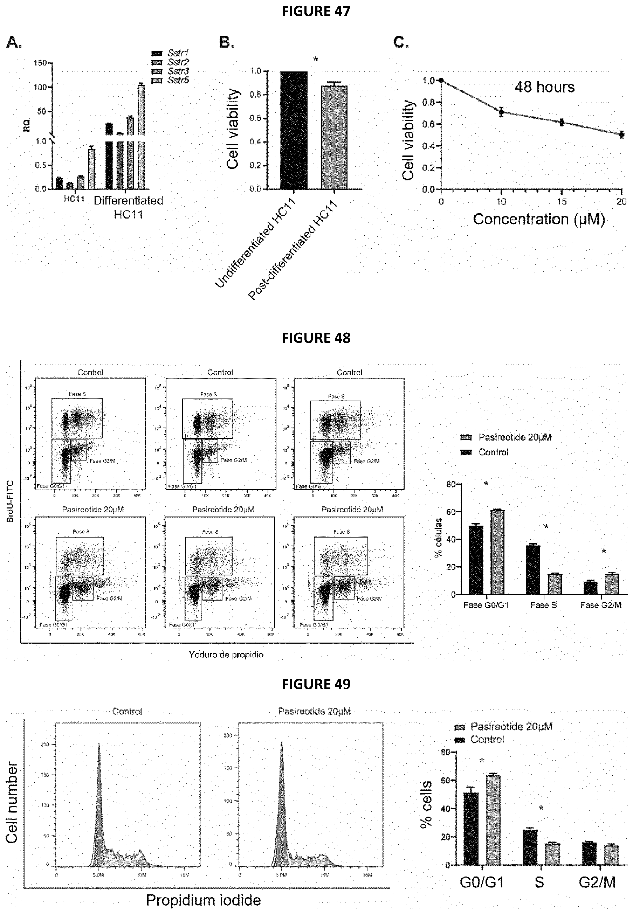

**Figure 47****. Analysis of cell viability in the HC11 line after lactogenic differentiation.** (A) Quantification of somatostatin receptors by QPCR in undifferentiated and differentiated HC11. (B) Comparison of baseline (untreated) viability between undifferentiated HC11 and post-differentiated HC11 cells by MTT. Mann-Whitney U test. (C) MTT assay in HC11 cells after loss of lactogenic differentiation, after 48 and 72 hours of pasireotide treatment.
**Figure 48****. Evaluation of proliferation and the different phases of the cell cycle in HC11 cells after treatment with pasireotide.** Assays with BrdU and evaluation by flow cytometry. Left. Dot plot representing control cells (top) and cells treated with 20 µM pasireotide (bottom). Right. Comparison of the proportions between control and treated cells in the different phases of the cell cycle. Mann-Whitney U test. The asterisk indicates that there was statistical significance (P<0.05).
**Figure 49****. Cell cycle analysis in the non-tumor line MCF10A. Propidium iodide assay and flow cytometric analysis.** Left. Plot of propidium iodide distribution in control and pasireotide (20 µM) treated cells. Right. Comparison between control and treated cells in the different phases of the cell cycle. Mann-Whitney U test. The asterisk indicates that there was statistical significance (P < 0.05).

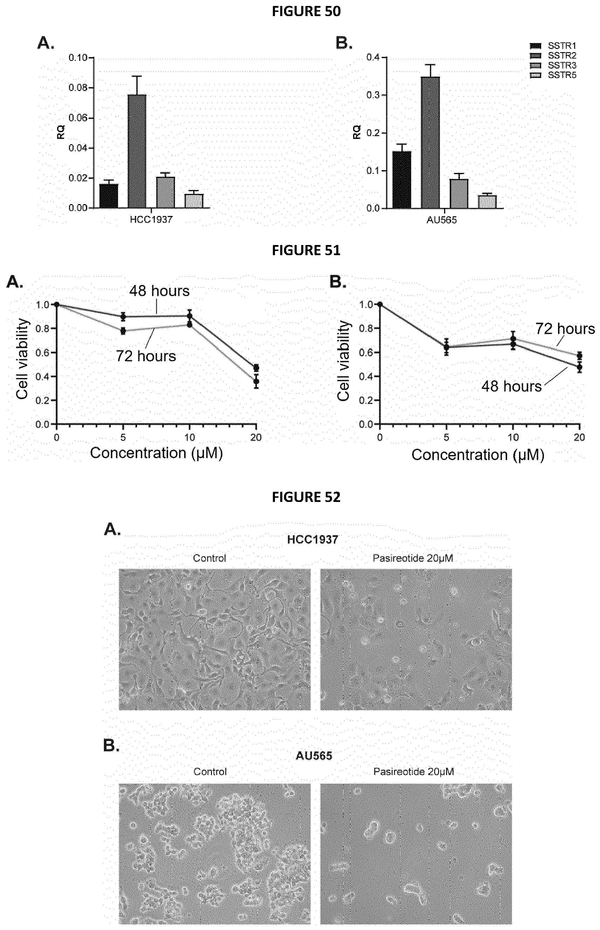

**Figure 50****. Quantification of the expression level of somatostatin receptors in tumor cell lines.** (A) Calculation of the RQ for SSTRs in HCC1937 cells. (B) Calculation of the RQ of SSTR receptors in AU565 cells. Human cutaneous epidermoid tumor RNA was used as positive control.
**Figure 51****. Viability study in breast cancer cell lines treated with pasireotide.** MTT assays in the presence of pasireotide at 5 µM, 10 µM and 20 µM. (A) HCC1937 cells with drug exposure for 48 and 72 hours. (B) AU565 cells at 48 and 72 hours of treatment.
**Figure 52****. Tumor cell lines treated with pasireotide.** (A) Untreated (left) and treated (right) HCC1937 cells. (B) Untreated (left) and treated (right) AU565 cells. In both cases, the cells were treated at a 20 µM concentration of pasireotide, for 48 hours. Images taken under a phase contrast microscope at 10X magnification.

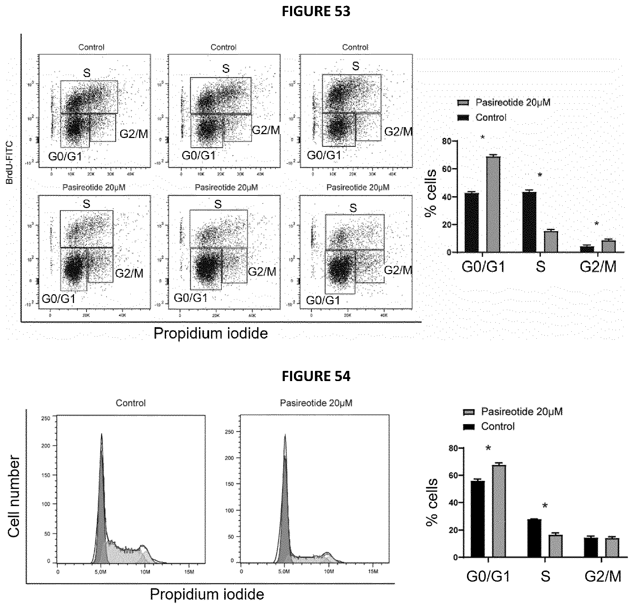

**Figure 53****. Study of the proliferation in the HCC1937 line treated with pasireotide. Assay with BrdU and evaluation by flow cytometry.** Left. Example of an experiment by dot plot depicting control cells (top) and cells treated with 20 µM pasireotide (bottom). Right. Comparison between control and treated cells in the different phases of the cell cycle. Mann-Whitney U test.
**Figure 54****. Cell cycle analysis in the AU565 tumor line. Propidium iodide assay and flow cytometric analysis.** Left. Plot of propidium iodide distribution in control and pasireotide (20 µM) treated cells. Right. Comparison between control and treated cells in the different phases of the cell cycle. Mann-Whitney U test.

### Detailed description of the invention

The present invention relates to pasireotide or a pharmaceutically acceptable salt or solvate thereof, for use in prevention or delay of appearance or development of breast cancer or post-pregnancy breast cancer in a female mammal. Analogously, the present invention relates to a method of prevention or delay of appearance or development of breast cancer or post-pregnancy breast cancer in a female mammal comprising administration thereto of pasireotide or a pharmaceutically acceptable salt or solvate thereof. Similarly, the present invention relates to the use of pasireotide or a pharmaceutically acceptable salt or solvate thereof for the manufacture of a medicament for prevention or delay of appearance or development of breast cancer or post-pregnancy breast cancer in a female mammal.

Pasireotide is a compound having the following chemical structure:

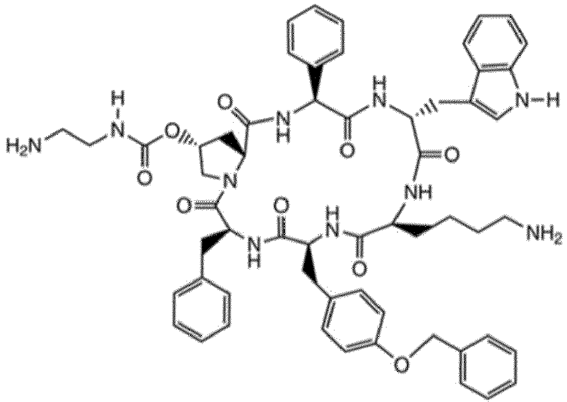

Pasireotide (CAS No: 396091-73-9) has the chemical name 1-[(6-allylergolin-8β-yl)-carbonyl]-1-[3-(dimethylamino) propyl]-3-ethylurea [IUPAC nomenclature: (6*aR*,9*R*,10*aR*)-*N*-[3-(dimethylamino)propyl]-*N*-(ethylcarbamoyl)-7-prop-2-enyl-6,6*a*,8,9,10,10*a*-hexahydro-4*H*-indolo[4,3-fg]quinoline-9-carboxamide], is synonymous with SOM230, and is sold under the brand name Signifor^{®}. Cabergoline also exists as the acetate salt (pasireotide acetate, CAS No: 396091-76-2), diaspartate salt (pasireotide diaspartate, CAS No: 820232-50-6) and pamoate salt (pasireotide pamoate, CAS No: 396091-79-5). Pasireotide pamoate has the following chemical structure: 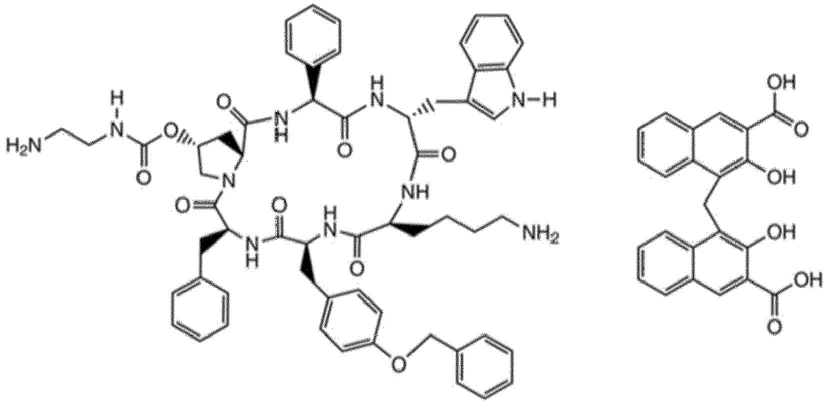 Pasireotide pamoate is synonymous with pasireotide LAR, SOM230 LAR and is sold under the brand name Signifor^{®} LAR. Note, however, that other long-acting release (LAR) forms of pasireotide exist, including that claimed in WO2022029782 A1. In a preferred embodiment of the present invention, said pasireotide or said salt or solvate thereof is selected from the group consisting of pasireotide, pasireotide, pasireotide acetate, pasireotide L-aspartate, pasireotide D-aspartate, pasireotide diaspartate, pasireotide aspartate-trifluoroacetate, pasireotide aspartate-trifluoroacetate and pasireotide pamoate, more preferably said pasireotide or said salt or solvate thereof is pasireotide pamoate or a solvate thereof.

Said solvate is preferably a pharmaceutically acceptable solvate. Said solvate may be a solvate of water (i.e. if crystalline, said co-crystal is a hydrate), a solvate of an organic solvent such as methanol, ethanol, dichloromethane, chloroform, acetone, acetonitrile or dimethylsulfoxide, a solvate or co-crystal of n-ethyl, n-propyl or n-butyl carboxylic acids. Said solvate is preferably a solvate of water. More preferably, said solvate is the monohydrate, dihydrate, trihydrate, tetrahydrate, pentahydrate or hexahydrate.

In the present invention, "prevention" refers to preventing appearance or development of breast cancer or post-pregnancy breast cancer, or embodiments thereof, in said female mammal. On the other hand, in the present invention, "delay" refers to delaying appearance or development of breast cancer or post-pregnancy breast cancer, or embodiments thereof, in said female mammal. Thus, delaying appearance or development of said breast cancer or said post-pregnancy breast cancer may involve, for example, slowing the rate at which said breast cancer or said post-pregnancy breast cancer develops in said female mammal. In another example delaying appearance or development of said breast cancer or said post-pregnancy breast cancer may preferably involve delaying metastasis (and hence dissemination) of said breast cancer or said post-pregnancy breast cancer.

Said breast cancer or post-pregnancy breast cancer, as defined in the present invention, is a proliferative disease of breast tissue, preferably a cancer of the breast tissue which exhibits anaplasia, invasiveness and/or metastasis. In a preferred embodiment of the present invention, said breast cancer is metastatic. Thus, said preferred embodiment of the present invention relates to pasireotide or a pharmaceutically acceptable salt or solvate thereof, for use in prevention or delay of appearance or development of metastatic breast cancer or metastatic post-pregnancy breast cancer in a female mammal. Analogously, a preferred embodiment of the method of the present invention relates to a method of prevention or delay of appearance or development of metastatic breast cancer or metastatic post-pregnancy breast cancer in a female mammal comprising administration thereto of pasireotide or a pharmaceutically acceptable salt or solvate thereof. Similarly, a preferred embodiment of the use of the present invention relates to use of pasireotide or a pharmaceutically acceptable salt or solvate thereof for the manufacture of a medicament for prevention or delay of appearance or development of metastatic breast cancer or metastatic post-pregnancy breast cancer in a female mammal. Even more preferably, said breast cancer or post-pregnancy breast cancer, as defined in the present invention, is a breast adenocarcinoma.

In another preferred embodiment of the present invention, said breast cancer exhibits cellular pleomorphism, more than 16 mitoses and no type of conserved structure. Pleomorphism is variability in the size and/or shape of cells and/or their nuclei, optionally also variability in the staining of cells and/or their nuclei. A conserved structure is that of normal (i.e. healthy, non-cancerous) breast tissue. Thus, said preferred embodiment of the present invention relates to pasireotide or a pharmaceutically acceptable salt or solvate thereof, for use in prevention or delay of appearance or development of breast cancer which exhibits cellular pleomorphism, more than 16 mitoses and no type of conserved structure, or post-pregnancy breast cancer which exhibits cellular pleomorphism, more than 16 mitoses and no type of conserved structure, in a female mammal. Analogously, a preferred embodiment of the method of the present invention relates to a method of prevention or delay of appearance or development breast cancer which exhibits cellular pleomorphism, more than 16 mitoses and no type of conserved structure, or post-pregnancy breast cancer which exhibits cellular pleomorphism, more than 16 mitoses and no type of conserved structure in a female mammal comprising administration thereto of pasireotide or a pharmaceutically acceptable salt or solvate thereof. Similarly, a preferred embodiment of the use of the present invention relates to use of pasireotide or a pharmaceutically acceptable salt or solvate thereof for the manufacture of a medicament for prevention or delay of appearance or development of breast cancer which exhibits cellular pleomorphism, more than 16 mitoses and no type of conserved structure, or post-pregnancy breast cancer which exhibits cellular pleomorphism, more than 16 mitoses and no type of conserved structure in a female mammal.

Said breast cancer is moreover selected from a cancer of the epithelial or mesenchymal tissues of the breast. Preferably, said breast cancer is a cancer of epithelial tissue of the breast. In a preferred embodiment of the present invention, said breast cancer is a cancer of ductal epithelial tissue. Thus, said preferred embodiment of the present invention relates to pasireotide or a pharmaceutically acceptable salt or solvate thereof, for use in prevention or delay of appearance or development of ductal epithelial breast cancer or ductal epithelial post-pregnancy breast cancer in a female mammal. Analogously, a preferred embodiment of the method of the present invention relates to a method of prevention or delay of appearance or development of ductal epithelial breast cancer or ductal epithelial post-pregnancy breast cancer in a female mammal comprising administration thereto of pasireotide or a pharmaceutically acceptable salt or solvate thereof. Similarly, a preferred embodiment of the use of the present invention relates to use of pasireotide or a pharmaceutically acceptable salt or solvate thereof for the manufacture of a medicament for prevention or delay of appearance or development of ductal epithelial breast cancer or ductal epithelial post-pregnancy breast cancer in a female mammal. Even more preferably, said breast cancer or post-pregnancy breast cancer, as defined in the present invention, is a cancer of luminal ductal epithelial tissue. Yet more preferably, said breast cancer or post-pregnancy breast cancer, as defined in the present invention, is a metastatic breast adenocarcinoma of ductal epithelial tissue.

Said female mammal, as defined in the present invention, is preferably a female human or a female domesticated mammal such as a canine, feline, bovine, ovine, porcine, equine, camelline, caprine or cervine. More preferably, said mammal is a human *(i.e.* a woman), dog (*i.e.* a bitch), cattle *(i.e.* a cow), sheep (i.e. a ewe), pig (i.e. a sow), horse (i.e. a mare), or camel (i.e. a camel cow), even more preferably a human or dog. In a preferred embodiment of the present invention, said female mammal is a woman.

Preferably, said female mammal expresses a somatostatin receptor. In a preferred embodiment of the present invention, said female animal expresses a somatostatin receptor in epithelial breast cells. In a more preferred embodiment of the present invention, said somatostatin receptor is encoded by a gene selected from the group consisting of: *Sstr1*, *Sstr2, Sstr3* and *Sstr5.*

Preferably, said female mammal has a mutation in a tumour suppressor gene and/or an oncogene. Preferably, said tumor suppressor gene is a breast cancer suppressor gene selected from the group consisting of: *Brca1, Brca2, P53 (TP53), CHK2, FANCD2, ATM, BRIP1, PALB2* (*FANCN*), *PTEN and STK11* and said oncogene is an epidermal growth factor receptor gene such as the *ErbB2 (HER2*/*neu)* gene. More preferably, said female mammal has a mutation in the *Brca1* gene, *Brca2* gene and/or the *P53* gene and/or the *ErbB2* gene. In a preferred embodiment of the present invention, said female mammal has a mutation in the *Brca1* gene and/or the *P53* gene and/or the *ErbB2* gene. Thus, said preferred embodiment of the present invention relates to pasireotide or a pharmaceutically acceptable salt or solvate thereof, for use in prevention or delay of appearance or development of breast cancer or post-pregnancy breast cancer in a female mammal, wherein said female mammal has a mutation in the *Brca1* gene and/or the *P53* gene and/or the *ErbB2* gene. Analogously, a preferred embodiment of the method of the present invention relates to a method of prevention or delay of appearance or development of breast cancer or post-pregnancy breast cancer in a female mammal comprising administration thereto of pasireotide or a pharmaceutically acceptable salt or solvate thereof, wherein said female mammal has a mutation in the *Brca1* gene and/or the *P53* gene and/or the *ErbB2* gene. Similarly, a preferred embodiment of the use of the present invention relates to use of pasireotide or a pharmaceutically acceptable salt or solvate thereof for the manufacture of a medicament for prevention or delay of appearance or development of breast cancer or post-pregnancy breast cancer in a female mammal, wherein said female mammal has a mutation in the *Brca1* gene and/or the *P53* gene and/or the *ErbB2* gene.

Thus, said breast cancer (including post-pregnancy breast cancer), as defined herein, may be either hereditary breast cancer or non-hereditary breast cancer. An example of hereditary breast cancer includes that caused by a mutation in the *Brca1* gene and/or the *P53* gene, while an example of non-hereditary breast cancer includes that caused by a mutation in the *ErbB2* gene.

As disclosed herein, a particularly preferred embodiment of the present invention relates to pasireotide or a pharmaceutically acceptable salt or solvate thereof, for use in prevention or delay of appearance or development of post-pregnancy breast cancer in a female mammal. Analogously, a particularly preferred embodiment of the method of the present invention relates to a method of prevention or delay of appearance or development of post-pregnancy breast cancer in a female mammal comprising administration thereto of pasireotide or a pharmaceutically acceptable salt or solvate thereof. Similarly, a particularly preferred embodiment of the use of present invention relates to use of pasireotide or a pharmaceutically acceptable salt or solvate thereof for the manufacture of a medicament for prevention or delay of appearance or development of post-pregnancy breast cancer in a female mammal. In said particularly preferred embodiments, any effect provided by pregnancy in prevention or delay of appearance or development of breast cancer in a female mammal is potentiated by pasireotide or a pharmaceutically acceptable salt or solvate thereof. Preferably said effect is synergic.

Said post-pregnancy breast cancer is any breast cancer that appears or develops following pregnancy in a female mammal, however when said female mammal is a parous woman, said post-pregnancy breast cancer preferably is that which is prevented or has delayed appearance or development up to 20 years after having given birth, more preferably up to 10 years after having given birth, even more preferably between 2 and 8 years after having given birth, still more preferably between 4 and 7 years after having given birth. Thus, the aforementioned particularly preferred embodiments relate to prevention or delay of appearance or development of any breast cancer in a female mammal following pregnancy, however when said female mammal is a parous woman, said post-pregnancy breast cancer preferably is that which is prevented or has delayed appearance or development up to 20 years after having given birth, more preferably up to 10 years after having given birth, even more preferably between 2 and 8 years after having given birth, still more preferably between 4 and 7 years after having given birth.

Said post-pregnancy breast cancer may be that which is prevented or has delayed appearance or development following a first, second, third, fourth, fifth or more pregnancies (and hence births), preferably following a first, second, third or fourth pregnancies, more preferably following first, second or third pregnancies.

Said post-pregnancy breast cancer preferably may be that which is prevented or has delayed appearance or development following weaning of the last offspring to have been born from the last pregnancy. More preferably, said post-pregnancy breast cancer may be that which is prevented or has delayed appearance or development in a female mammal who has weaned their offspring. Thus, a preferred embodiment of the present invention relates to pasireotide or a pharmaceutically acceptable salt or solvate thereof, for use in prevention or delay of appearance or development of post-pregnancy breast cancer in a female mammal, wherein said female mammal has weaned their offspring. Analogously, a preferred embodiment of the method of the present invention relates to a method of prevention or delay of appearance or development of post-pregnancy breast cancer in a female mammal comprising administration thereto of pasireotide or a pharmaceutically acceptable salt or solvate thereof, wherein said female mammal has weaned their offspring. Similarly, a preferred embodiment of the use of the present invention relates to use of pasireotide or a pharmaceutically acceptable salt or solvate thereof for the manufacture of a medicament for prevention or delay of appearance or development of post-pregnancy breast cancer in a female mammal, wherein said female mammal has weaned their offspring.

Even more preferably, said post-pregnancy breast cancer may be that which is prevented or has delayed appearance or development once lactation or milk production has begun to cease or has fully ceased. Still more preferably, said post-pregnancy breast cancer may be that which is prevented or has delayed appearance or development during or after post-lactational involution. Thus, a preferred embodiment of the present invention relates to pasireotide or a pharmaceutically acceptable salt or solvate thereof, for use in prevention or delay of appearance or development of post-pregnancy breast cancer in a female mammal, wherein said pasireotide or said salt or solvate is administered to said female mammal during and/or after post-lactational involution, more preferably during post-lactational involution. Analogously, another preferred embodiment of the method of the present invention relates to a method of prevention or delay of appearance or development of post-pregnancy breast cancer in a female mammal comprising administration thereto of pasireotide or a pharmaceutically acceptable salt or solvate thereof, wherein said pasireotide or said salt or solvate is administered to said female mammal during and/or after post-lactational involution, more preferably during post-lactational involution. Similarly, a preferred embodiment of the use of the present invention relates to use of pasireotide or a pharmaceutically acceptable salt or solvate thereof for the manufacture of a medicament for prevention or delay of appearance or development of post-pregnancy breast cancer in a female mammal, wherein said pasireotide or said salt or solvate is administered to said female mammal during and/or after post-lactational involution, more preferably during post-lactational involution.

When said female mammal is a parous woman, said woman is preferably ≥ 25 years old when having given birth, more preferably when having first given birth. In a preferred embodiment of the present invention, said woman is ≥ 30 years old when having given birth, more preferably said woman is ≥ 35 years old when having given birth, even more preferably wherein "having given birth" means "having first given birth". Thus, said preferred embodiment of the present invention relates to pasireotide or a pharmaceutically acceptable salt or solvate thereof, for use in prevention or delay of appearance or development of post-pregnancy breast cancer in a woman, wherein said woman is ≥ 30 years old when having given birth, more preferably ≥ 35 years old when having given birth, even more preferably wherein "having given birth" means "having first given birth". Analogously, a preferred embodiment of the method of the present invention relates to a method of prevention or delay of appearance or development of post-pregnancy breast cancer in a female mammal comprising administration thereto of pasireotide or a pharmaceutically acceptable salt or solvate thereof, wherein said woman is ≥ 30 years old when having given birth, more preferably ≥ 35 years old when having given birth, even more preferably wherein "having given birth" means "having first given birth". Similarly, a preferred embodiment of the use of the present invention relates to use of pasireotide or a pharmaceutically acceptable salt or solvate thereof for the manufacture of a medicament for prevention or delay of appearance or development of post-pregnancy breast cancer in a female mammal, wherein said woman is ≥ 30 years old when having given birth, more preferably ≥ 35 years old when having given birth, even more preferably wherein "having given birth" means "having first given birth".

In another embodiment when said female mammal is a parous woman, said woman was preferably pregnant for more than 32 weeks during at least one pregnancy, more preferably during each pregnancy. In a preferred embodiment of the present invention, said female mammal is a parous woman who was pregnant for more than 34 weeks. Thus, said preferred embodiment of the present invention relates to pasireotide or a pharmaceutically acceptable salt or solvate thereof, for use in prevention or delay of appearance or development of post-pregnancy breast cancer in a woman, wherein said woman was pregnant for more than 34 weeks. Analogously, a preferred embodiment of the method of the present invention relates to a method of prevention or delay of appearance or development of post-pregnancy breast cancer in a female mammal comprising administration thereto of pasireotide or a pharmaceutically acceptable salt or solvate thereof, wherein said woman was pregnant for more than 34 weeks. Similarly, a preferred embodiment of the use of the present invention relates to use of pasireotide or a pharmaceutically acceptable salt or solvate thereof for the manufacture of a medicament for prevention or delay of appearance or development of post-pregnancy breast cancer in a female mammal, wherein said woman was pregnant for more than 34 weeks. More preferably, when said female mammal is a parous woman, said woman was preferably pregnant for more than 36 weeks during at least one pregnancy, yet more preferably during each pregnancy.

### Examples

The following examples are for illustrative purposes and are not intended to limit the claimed invention.

### Material and methods

### Mice

### K14Cre; Brca1^{F/}^{F}; P53^{F/F} mice

This study model used is a genetically modified mouse, generated by Jos Jonkers' group at the *Netherland Cancer Institute* and generously provided to our group. It is a conditional double knock-out (KO) for *Brca1* and *P53* expressing Cre-recombinase under the promoter of the *cytokeratin-14 (K14)* gene; model named *K14Cre; Brca1*^{*F*/}*^{F}; P53*^{*F*/*F*} (*Brca1* or KO, henceforth). These mice develop breast tumors with 100% penetrance (although it depends on whether they develop skin cancer first and must be sacrificed) and a median age of 7 months. These tumors are similar to human tumors with *BRCA1* mutations, as they are basal-like and lose *P53.* Since Cre-recombinase under the *K14* gene promoter is expressed in mammary cells and other epithelial cells, these mice develop squamous carcinoma-type skin tumors. The cohort of mice used in this study is in FVB/N genetic background. Subsequently, the same groups of animals were generated, and mammary glands were collected at 60 days post-weaning (in lactating multiparous, without, and with treatment) (N= 5 mice per group). Each figure indicates the number of mice used in the chemoprevention experiments.

### FVB/N - Tg(MMTV-Neu)202Mul/J mice

Transgenic mice overexpressing the ErbB2/Neu protooncogene under the Mouse Mammary Tumor Virus (MMTV) promoter (LTR region) and were obtained from the Jackson laboratory. The transgene originated from the wild sequence of the rat *ErbB2*/*Neu* protooncogene. Also, alternative splicing sequences and polyadenylation sites from the SV-40 polyomavirus were included to provide higher transcript stability. The transgene does not produce phenotypic changes in male mice and is expressed at low levels in the normal mammary epithelium, thymus, spleen, salivary gland, lungs, and testicles. In female mice with FVB/N genetic background, the transgene expression induces the development of focal adenocarcinomas in the mammary gland surrounded by hyperplastic breast tissue with a latency period between eight and twelve months. In addition, these mice develop multifocal lung metastases with an incidence of 70% from nine months of age. The LTR promoter of MMTV contains response elements for glucocorticoids, androgens, and progesterone. Hormones highly regulate the expression of genes adjacent to this promoter, and there is an increase in the expression of ErbB2 during pregnancy, favoring the development of more aggressive breast tumors. Thus, this model is not appropriate to study pregnancy protection of breast cancer. We will refer to it as model MMTV-ErbB2/Neu. Each figure indicates the number of mice used in the different experiments.

### K14Cre; Brca1^{F/}^{F}; P53^{F/F} ROSA26 mice

The mouse model used for cytometry studies was obtained by crossing the conditional *K14Cre; Brca1*^{*F*/}*^{F}; P53*^{*F*/*F*}model *with* a model expressing red fluorescent protein (RFP) under the ROSA26 locus promoter. This promoter has a stop codon flanked by *LoxP* sites that recognizes Cre-recombinase. Thus, cells that lose *Brca1* and *P53* through the action of Cre-recombinase also express RFP and emit red fluorescence. The mouse was obtained from the European Mutant Mouse Archive (EMMA) and generated by Hans Jörg Fehling's group at the Institute of Immunology, Clinical University of Ulm, Germany. Between 6 and 11 mice in each study group were used

### Preparation and administration of pasireotide

Signifor-LAR (Long-Acting Release) (pasireotide-LAR) was used in the examples of the invention. Pasireotide was administered subcutaneously at a 40 mg/kg concentration every 28 days (20 mg/ml) (Walls *et al.*, 2016). The drug was diluted in 45 mg of D-mannitol (#M4125, Sigma-Aldrich), 7 mg of sodium carboxymethylcellulose (#21902-100G, Sigma-Aldrich), and 4 mg of poloxamer 188 (Kolliphor^{®} P 188, #15759-1KG, Sigma-Aldrich) in turn dissolved in distilled water. To prepare the diluent, boiling distilled water was used, and sodium carboxymethylcellulose was gradually added as it was diluted. Once the sodium carboxymethylcellulose was dissolved, D-mannitol and poloxamer 188 were added to the stirring mix. Subsequently, the diluent was aliquoted in 1.5 ml tubes (Eppendorf) and stored at 4°C. Before administering the drug, the mice were weighed to calculate the drug dose. The drug was administered subcutaneously with a syringe (ICO plus 3 Tuberculin 0.5 x 16, #N14085, Peroxfarma) under anesthesia with isoflurane (Vetflurane^{®}, #575837-4, Virbac).

### Evaluation of breast cancer pathophenotypes

Different pathophenotypes were considered to characterize breast cancer susceptibility and progression in the *Brca1* KO mouse cohort. These were: (i) *tumor latency,* which is defined as the time from mouse birth to the appearance of the first breast tumor; (ii) *tumor incidence,* which is the percentage of mice developing tumor relative to the total in that group; and (iii) *tumor multiplicity,* which is the percentage of mice that developed more than one tumor relative to the total number of mice that developed a tumor. Brca1- and p53-deficient females from the different study groups were checked and palpated once a week to assess these parameters. Animals were sacrificed when they showed signs of disease or tumors reached 2.5 cm in greatest diameter. Tumor volume was estimated with the classic formula: (largest diameter x most minor diameter ²) / 2. The straight-line slope formed by successive tumor volume measurements represents the tumor growth rate. Lung metastases were counted by a bench magnifier (Leica). The mouse study was approved by the Bioethics Committee of the University of Salamanca.

### Embedding of tissues in paraffin

For further studies, each mouse's left inguinal mammary gland was embedded in paraffin. Once removed from the animals, the breasts were fixed in 4% paraformaldehyde (Scharlau FO) for 24 hours and then kept in 70% ethanol at a temperature of 4°C until they were processed in *the Comparative Molecular Pathology Core* of our Centre. The tissues were dehydrated by three 1-hour incubations in increasing ethanol concentrations (60%, 80%, and 100%). Three further 1-hour incubations in xylol were performed to replace the dehydrating agent with a substance miscible in paraffin. Finally, three further incubations of 1 hour and 20 minutes in liquid paraffin were performed, after which blocks were made in a *paraffin embedding station.* When the paraffin blocks were utterly cooled, 2 µm thick cuts were made using a rotary microtome *(Leica RM2255).*

### Hematoxylin-eosin staining

Staining with hematoxylin and eosin was carried out at the *Comparative Molecular Pathology* Core of the Salamanca *Cancer Research Centre* using a manual staining battery following the processes: dewaxing in xylol, rehydration with ethanol at different levels, hematoxylin and eosin staining, dehydration with ethanol and rinsing. Once the process was completed, the samples were mounted by adding a drop of non-aqueous fixative DPX, and the coverslip was placed.

### Assessment of proliferation by detecting of Ki-67 by immunohistochemistry

Staining for Ki67 was performed at the Comparative Molecular Pathology Core of the Institute of Molecular and Cellular Biology of Cancer (IBMCC) in Salamanca. Slices of the sample were made at 3 µm thickness. The sections were fished on slides specially treated for this technique *(Leica BOND Plus Slides, #S21.2113.A, Leica).* The staining process was carried out using the Roche Discovery ULTRA research instrument. The protocol performed by the apparatus is as follows: deparaffinization of the sample by heating the slide to 69°C; cell conditioning consisting of epitope unmasking by incubating the sample in a TRIS EDTA pH8 buffer at 100°C for 32 minutes. The primary antibody (Rabbit Anti-Human Ki-67 Monoclonal Antibody (Clone SP6), *#MAD-020310Q, Master Diagnostica)* was then manually applied at a 1:50 dilution and incubated at 37°C for 60 minutes. After incubation of the primary antibody, two washes with HRP buffer were performed. Subsequently, the sample was incubated with the pre-diluted secondary antibody (OmniMap anti-Rb HRP, *#05269679001, Roche)* conjugated with horseradish peroxidase for 16 minutes and developed with diaminobenzidine (DAB). Finally, the slide was counterstained with hematoxylin and wiped clean. Finally, the coverslip was manually mounted by applying a drop of DPX resin.

### Quantification of ductal area in the mammary gland

The Ductal area was assessed using free *ImageJ software.* Ductal area was defined as the surface area occupied by the ductal epithelium and the surrounding fibrous tissue stroma, excluding the area of the tubular lumen. To assess ductal areas, ten photos were taken at 10X magnification of the mammary gland surface of each of the study animals, five photos in the distal and five in the proximal area of the mammary gland. The photos were taken randomly in each of these areas of the samples stained with hematoxylin-eosin, using a *Leica DM1000* light microscope. Evaluation of the ductal area percentages showed a gradient from more extensive to lesser involution from the proximal to the distal zone. More adipose tissue and less ductal tissue were observed in the proximal zone than in the distal zone, and vice versa (Figure 57). Therefore, we only took the values from the distal zone for the analyses.

Quantification was expressed as a percentage of the total area. The percentage of the ductal area in the mammary gland was defined as the total ductal area divided by the total area of the mammary gland present in each photo (multiplied by one hundred to express as percentages).

### Quantification of cells positive to immunohistochemistry reactions

*The Leica Application Suite (LAS)* V3.7 software was used to quantify Ki-67 positive cells. Eight photos were taken at 20X of the mammary gland with the *Leica DM1000* light microscope. Parameters were then defined for the program to distinguish between positive and negative cells. The portion of interest (ductal epithelium) was manually defined; then, the program automatically provided the number of positive and negative cells in the selected region. In both cases, the percentage of positive cells was defined as the number of positive cells divided by the number of total cells (positive plus negative).

### Flow Cytometry

After being removed, the mouse mammary glands were incubated in a 50 ml tube containing 9 ml of medium (Gibco DMEM/F-12, #11554546, Fisher Scientific), 500 µl collagenase, and 500 µl hyaluronidase, at 37°C for 16 h. After incubation, the aggregated tissue debris was disintegrated by pipetting for approximately 30 seconds and centrifuged for 5 min at 350 g at room temperature. After incubation, the aggregated tissue debris was disintegrated by pipetting for approximately 30 seconds, and the sample was centrifuged for 5 minutes at 350 g at room temperature. The supernatant was discarded, and the pellet formed was resuspended in 2 ml of ACK buffer (Gibco ACK Lysing Buffer, #A10492-01, Fisher Scientific) to lyse the erythrocytes present in the sample. The disaggregated sample was incubated in ACK buffer for 5 minutes at room temperature and then centrifuged at 350 g for 5 minutes. After centrifugation, the supernatant was discarded, and 1 ml of trypsin was added at 37°C. The pellet was resuspended by pipetting for 2 min. Next, 10 ml of saline (Gibco HBSS, *#14025-092, Fisher Scientific)* supplemented with 10 mM HEPES buffer and fetal bovine serum was added and centrifuged for 5 minutes at 350 g at room temperature. The supernatant was then discarded, and 1 ml of saline buffer (hereafter called SB) and 100 µl of warm DNase were added, pipetted carefully for 1 minute, and 5 ml of cold SB was added to stop the action of DNase. The mixture was filtered through a 40 µm filter and washed with 5 ml of cold SB. The mixture was centrifuged for 5 minutes at 350 g at 4°C, and the supernatant was removed after centrifugation. The mixture was then resuspended in 5 ml of cold SB, and the mixture was distributed into 35 µm filter cytometry tubes. 4/5 of the sample was used for immunolabelling, and the remaining 1/5 was used as an unlabelled control. The supernatant was centrifuged for 5 minutes at 1200 rpm at 4°C, and the supernatant was decanted after centrifugation. 52 µl of blocking solution, consisting of 50 µl of SB and 2 µl of anti-CD16/CD32 antibody, was added, gently vortexed, and incubated for 10 minutes at 4°C. Then 50 µl of the immunolabelling solution consisting of the following antibodies: CD45 FITC, CD140 FITC, CD31 FITC, Ter119 FITC, CD49f APC, CD29 APC- Cy7, CD61 BV421, Sca1 PerCP-Cy5, EpCAM PE-Cy7, e506, was added to the staining tube and 50 µl of SB to the control tube. After incubation for 30 minutes and washing, the samples were acquired with the LSR Fortessa X-20 Cell Analyser cytometer (BD Biosciences). Analyses were performed with *Flow Jo* software.

### Cell lines

To evaluate the effects of pasireotide in vitro, four cell lines were used, three of them obtained from the American Type Culture Collection (ATCC, Rockville, Maryland); and a fourth, the HC11 line, was a donation from Dr. Lluis Montoliu of the National Center for Biotechnology (CNB) in Madrid.

### HC11 cell line

This line is derived from normal mouse mammary epithelium. It is a prolactin-sensitive clone derived from an epithelial cell line called COMMA 1D, originating from a pregnant BALB/c female mouse. HC11 cells respond to a cocktail of hormones that induce lactogenic differentiation, including: prolactin, insulin, and dexamethasone or hydrocortisone). In this process of lactogenic differentiation, the cells show vesicles in the cytoplasm, rich in proteinaceous material and express milk proteins such as beta-casein. HC11 cells are commonly used as an in vitro model to study lactogenic differentiation and postlactational involution.

Line HC11 grows in adhesion and monolayer with RPMI-1640 (#21875034, ThermoFisher Scientific), supplemented with 10% fetal bovine serum (Gibco^{™} Fetal Bovine Serum, #26140079, Fisher Scientific), 1% penicillin/streptomycin (10,000 U/mL), 200 mM glutamine (Gibco^{™} L-glutamine, #25030081, Fisher Scientific), 5 µg/mL insulin (#I0516-5ML, Sigma-Aldrich), and 10 ng/mL epidermal growth factor (#RP8661, ThermoFisher Scientific). To induce lactogenic differentiation, the old growth medium was replaced by differentiation medium, consisting of RPMI-1640, supplemented with 10% fetal bovine serum, 1% penicillin/streptomycin, 5 µg/ml insulin, 5 µg of prolactin (Recombinant Murine Prolactin, #315-16, PeproTech) and 1 µg of hydrocortisone (#H0888, Sigma-Aldrich). Cells were kept for three days in this differentiation medium.

### MCF10A cell line

It is a human cell line obtained from non-tumorous mammary epithelium. These cells were isolated from a 36-year-old Caucasian woman with no family history of breast cancer and a benign breast fibrocystic lesion.

The growth of this cell line is in adhesion and monolayer. They were cultured in DMEM/F-12 medium (#11554546, ThermoFisher Scientific), supplemented with 5% horse serum (Gibco Horse serum, heat inactivated, New Zealand origin, #26050088, ThermoFisher Scientific), 1% penicillin/ streptomycin, 200 mM glutamine (Gibco^{™} L-glutamine, #25030081, Fisher Scientific), 0.5 µg/mL hydrocortisone (#H0888, Sigma-Aldrich), 10 µg/mL insulin (#I0516-5ML, Sigma-Aldrich), 20 ng/mL epidermal growth factor (EGF) (#RP8661, ThermoFisher Scientific), and 100 ng/mL cholera toxin (Cholera Toxin from Vibrio cholera, #C88052, Sigma-Aldrich).

### HCC1937 cell line

This cell line was isolated from a grade III infiltrating ductal carcinoma in a 23-year-old Caucasian female who carried a germline mutation in the BRCA1 gene. The cells were described as "triple negative". The line has homozygous mutations in 5382C of BRCA1 and in the RB1 gene. In addition, it presents an acquired mutation in the TP53 gene, with loss of the two wild type alleles, which causes its loss of expression. Also, it has an acquired homozygous deletion in the PTEN gene and a loss of heterozygosity at multiple loci implicated in the pathogenesis of breast cancer.

These cells are adherent and have an epithelial morphology. They were cultured in RPMI-1640 (#21875034, ThermoFisher Scientific), supplemented with 10% fetal bovine serum (Gibco^{™} Fetal Bovine Serum, #26140079, Fisher Scientific), 1% penicillin/streptomycin (#I0516-5ML, Sigma -Aldrich) and 200 mM glutamine (Gibco^{™} L-glutamine, #25030081, Fisher Scientific).

### AU565 cell line

Cell line isolated from the pleural effusion of a 43-year-old Caucasian patient with adenocarcinoma of the breast, who was treated with radiotherapy, steroids, cyclophosphamide, and 5-fluorouracil. From this same patient, the SK-BR-3 line (which is not part of this study) was isolated. AU565 cells have an overexpression and amplification of the HER2/NEU oncogene and have expressed the oncogenic version of HER3, HER4 and TP53.

The cells show adherent growth, the culture medium used was RPMI-1640 (#21875034, ThermoFisher Scientific), supplemented with 10% fetal bovine serum (Gibco^{™} Fetal Bovine Serum, #26140079, Fisher Scientific), 1% penicillin/streptomycin (#I0516-5ML, Sigma-Aldrich) and 200 mM glutamine (Gibco^{™} L-glutamine, #25030081, Fisher Scientific).

### Statistical analysis

Comparison of time variables was made with Kaplan-Meier curves and the significance level with the Log-Rank test. The Chi-square test or Fisher's exact test was used to compare proportions. The non-parametric Mann Whitney U-test evaluated phenotype differences between two groups since the samples did not follow a normal distribution. Normality was assessed by the Kolmogorov-Smirnov test. The Kruskal-Wallis test for non-normal populations was used when more than two groups were compared. After the overall comparison, a *post-hoc* test (usually Dunn's test) identified the groups with statistically significant differences. Correlation between continuous variables was studied using Pearson's test if the variables followed a normal distribution. Otherwise, Spearman's test was used. The significance level was set at a P ≤ 0.05. The statistical programs used to perform the tests described were *GraphPad Prism5* and *JMP 12.*

**Example 1. Evaluation of chemoprevention: chemoprevention with pasireotide increases resistance to mammary cancer in nulliparous mice overexpressing ErbB2/Neu protooncogene** First, we evaluated whether the antiproliferative effect of pasireotide could protect against mammary cancer in nulliparous mice. Mice overexpressing the *ErbB2*/*Neu* protooncogene (Neu+ mice hereafter) under the MMTV promoter were used. These mice develop breast cancer with 100% penetrance after eight months of age.

Thirty-two Neu+ female mice from the seventh week of age were treated with pasireotide-LAR subcutaneously once a month at the established dose. Tumor susceptibility and evolution were compared with 32 Neu+ control female mice. A statistically significant increase in tumor latency was observed in treated mice (p = 0.0069) (Figure 1A)

No differences in tumor incidence (percentage of mice that developed mammary tumor) were observed (Figure 1B). However, differences were observed in multiplicity (p = 0.0385) (understood as the percentage of mice with breast cancer that developed two or more tumors), as well as in the number of tumors, so treated mice significantly develop fewer tumors than the untreated ones (p = 0.0007) (Figure 1C, D).

In conclusion, pasireotide increased resistance to breast cancer in nulliparous Neu+ mice. The tumors appeared later (more latency), and there were fewer tumors per mouse (multiplicity and number). However, the percentage of mice that develop breast cancer does not decrease (incidence).

### Example 2. Assessment of tumor evolution: pasireotide treatment decreases lung metastasis in ErbB2/Neu+ nulliparous mice with breast cancer

Mice continued to be treated with one dose of pasireotide per month at the same dose to assess the effect of pasireotide on the progression of mammary tumors. No differences were observed in the duration of the disease, understood as the time from the appearance of the tumor until the mouse is sacrificed to reach the tumor size indicated in the Methods section (1.5 cm) (Figure 2A). No differences were observed in the rate of tumor growth (Figure 2B). In contrast, significant differences were observed in survival (P=0.029) (Figure 2C), which is attributed to the observed increase in tumor latency (notice that survival is the sum of latency and duration of the disease).

Interestingly, there was a statistically significant decrease in the incidence of metastases, defined as the percentage of tumor-bearing mice that developed metastases (P=0.0089) (Figure 2D). However, no differences were found in the multiplicity of metastases, defined as the percentage of mice with metastases that had two or more lung hits (Figure 2E).

In conclusion, pasireotide decreases the incidence of lung metastases in ErbB2/Neu+ mice with breast tumors. This result could be of interest to use pasireotide as a drug for secondary prophylaxis in breast cancer patients.

### Example 3. Evaluation of chemoprevention: pasireotide treatment increases resistance to breast cancer in Brca1/P53 deficient nulliparous mice

Subsequently, we evaluated the chemopreventive effect of pasireotide against breast cancer in another mouse model of breast cancer, mice deficient in *Brca1*/*P53.* These mice develop triple-negative basal breast cancer with 100% penetrance at seven months. These mice also develop squamous cell skin cancer due to the loss of *Brca1* and *P53* in the skin.

Thirty-five female *Brca1*/*P53* deficient mice from the seventh week were treated with pasireotide once a month at the used dose. Tumor susceptibility and progression were compared with 33 control female *Brca1*/*P53* untreated mice. A statistically significant increase in tumor latency was observed in treated mice (p = 0.044) (Figure 3A).

A decrease in tumor incidence was observed; that is, the percentage of mice that developed mammary tumors in treated mice was significantly lower (p=0.027; 88.7% (42/35) versus 66.7% (22/33)) (Figure 3B). No differences were found in either tumor multiplicity or the number of tumors (Figure 3C, D). The fact that there was a decrease in tumor incidence does not mean that the development of breast cancer was wholly inhibited in these mice. It means that fewer mice in that period developed mammary tumors. It must be considered that these mice die fundamentally from skin tumors that require the sacrifice of the mouse when it reaches 1 cm in diameter. If the skin tumor could have been inhibited, it cannot be ruled out that all of them would have developed breast cancer in the longer term. Even so, the result of the lower incidence of breast cancer for that study period is remarkable.

In conclusion, pasireotide increases resistance to breast cancer in nulliparous *Brca1*/*P53* deficient mice. The tumors appear later (more latency), and a lower percentage of mice develop it (incidence). So pasireotide could be used as a chemoprevention strategy against breast cancer.

### Example 4. Evaluation of tumor evolution in Brca1/P53 deficient nulliparous mice with breast cancer treated with pasireotide

Subsequently, we evaluated the evolution of breast cancer by continuing with the same treatment regimen of a monthly dose. No differences were observed in the duration of the disease (Figure 4A). Differences were observed in overall survival with a statistical trend (P=0.051) (Figure 4B). Although, this is attributed to the observed effect on the increase in latency. A statistically significant decrease in growth speed was observed (p= 0.038) (Figure 4C), which, despite this, had no impact on the duration of the disease.

No statistical differences were observed in the incidence of metastases, nor in the multiplicity regarding metastases. However, the number of mice with metastases was deficient in making conclusions (Figure 4 D, E). Although it must be considered that this model does not have well-characterized distant dissemination, we found pulmonary epithelial impacts of small size, and that could come from both breast and skin tumors.

In conclusion, pasireotide treatment only affected the tumor growth rate deficient *Brca1*/*P53* nulliparous mice

### Example 5. Histopathological evaluation of tumors in nulliparous mice treated with pasireotide compared to untreated mice

Breast tumors developed by pasireotide-treated nulliparous mice were then evaluated histologically. As seen previously, the tumors generated by the group of nulliparous without treatment, in 86.2% of the cases, were diagnosed as infiltrating ductal carcinoma of the breast, being poorly differentiated or undifferentiated in 41.2% of the cases. In the group of nulliparous treated with somatostatin analog, 95.2% of the tumors were infiltrating ductal carcinomas, which was not statistically significant compared to untreated nulliparous (P = 0.3830) (Figure 5E). In addition, 71.4% of them presented a good to moderate differentiation (P = 0.0333) (Figure 5F).

Thus, at the histopathological level, treatment with pasireotide in Brca1/P53-deficient nulliparous mice was associated with less aggressive breast cancer, with greater differentiation at the histopathological level.

### Example 6. Study of the effect of pasireotide on the susceptibility and evolution of breast cancer in nulliparous mice with overexpression of Erbb2/Neu

Then, the protective effect of pasireotide against breast cancer was evaluated in another mouse model with increased susceptibility to it. In this case, transgenic mice overexpressing the ErbB2/Neu protooncogene under the MMTV promoter (hereinafter referred to as ErbB2/Neu) were used. These mice develop breast cancer with 100% penetrance. The study was only carried out in nulliparous mice, since, as indicated in the methodology section, pregnancy induces the expression of the transgene regulated by MMTV and artificially gives rise to a tumor disease with lower latency and greater aggressiveness.

As a previous step to the study, as was done in the groups of Brca1/P53 mice. The expression of somatostatin receptors in the ErbB2/Neu overexpression mouse line was evaluated. Expression of the four receptors evaluated was observed, with notable values, higher than those obtained in the positive control (mouse brain), the receptors Sstr1 and Sstr5 (Figure 6).

### Example 7. Effect of pasireotide on breast cancer prevention in ErbB2/Neu nulliparous mice

In this study, 32 ErbB2/Neu nulliparous females were treated with pasireotide, from the eighth week of age, at the established dose and regimen. The susceptibility and subsequent tumor evolution of the treated group were compared with that of a control group made up of 28 ErbB2/Neu nulliparous females, who did not receive treatment.

A statistically significant increase in tumor latency was observed in the group of mice that received the treatment (P = 0.0069) (Figure 7A). No differences in tumor incidence were observed, as 100% of the mice developed tumor despite treatment (Figure 7B). In contrast, statistically significant differences were observed in tumor multiplicity (P = 0.0385) (Figure 7C). Thus, of the 28 control females, 24 of them generated more than one tumor (83.3%); on the contrary, in the treated group, of the 32 females, 20 generated two or more tumors (62.5%). Regarding the number of tumors, it was also observed that those animals that received treatment generated fewer mammary tumors on average (P = 0.0007) (Figure 7D).

Therefore, pasireotide treatment in ErbB2/Neu nulliparous mice exerted partial protection against breast cancer in terms of tumor latency, multiplicity and number of tumors, compared to untreated mice. However, in the end, 100% of the mice developed a mammary tumor.

### Example 8. Evaluation of the evolution of the disease in treated ErbB2/Neu nulliparous mice

To evaluate the effect of pasireotide on the progression of mammary tumors, the mice continued their treatment regimen, with the dose of pasireotide established every 28 days. No statistically significant differences were observed in the duration of the disease between treated animals and the control group (P = 0.1337) (Figure 8A). In contrast, animals that received treatment showed a significantly increased survival (P = 0.0294) (Figure 8B). This was attributed to increased tumor latency in this group. Tumor growth rate did not change with treatment (P = 0.9967) (Figure 8C). However, a statistically significant decrease in the incidence of metastases was observed in treated compared to untreated animals (P = 0.0113) (Figure 24D); no change in multiplicity (P = 0.8042) (Figure 8E).

In summary, pasireotide treatment of breast cancer in ErbB2/Neu mice was associated with slightly less aggressive disease; because it increased survival and decreased the ability to spread at a distance in treated mice.

### Example 9. Histopathological evaluation of tumors in ErbB2/Neu nulliparous mice treated with pasireotide

Afterwards, tumor disease was evaluated at the histopathological level in 10 cases from each of the study groups. The tumors generated by the ErbB2/Neu transgenic mice were in all cases infiltrating ductal adenocarcinomas, with little cell differentiation, high mitotic index and few areas of apoptosis (Figure 9A-D).

No notable histopathological differences were observed in the tumors developed by ErbB2/Neu nulliparous mice, treated and not treated with pasireotide (Figure 9E-F).

### Example 10. Evaluation of the model: pregnancy increases resistance to breast cancer in the Brca1/P53 deficient mouse model

Early and repeated pregnancy is one of the most resistant factors that induce breast cancer. Therefore, we previously evaluated the extent to which pregnancy induces resistance to breast cancer in the mouse model deficient in *Brca1*/*P53* in the mammary gland. The mice endured two rounds of pregnancy and provided breastfeeding, and latency and tumor incidence were compared in nulliparous and multiparous animals. Indeed, parous mice were more resistant to developing breast cancer (Figure 10).

### Example 11. Evaluation of chemoprevention: pasireotide potentiates the protective effect of pregnancy in the Brca1/P53 deficient mouse model

It has been described that pregnancy would induce epigenomic changes in the mammary gland epithelium and changes in the transcriptomic signatures and particularly in proliferation affecting TGFbeta, P27, Areg and Igf1 genes among others. Our working hypothesis is that if the antiproliferative effect of pregnancy is enhanced with drugs such as pasireotide, this could enhance the protective effect of pregnancy against breast cancer.

Therefore, we evaluated the effect of pasireotide-LAR on breast cancer susceptibility in *Brca1*/*P53-*deficient mice after pregnancy. Thus, pasireotide-LAR was administered after the second pregnancy and weaning on day 7 of post-weaning, and then once a month. The global comparison among groups showed statistically significant differences in tumor latency (P<0.0001) (Figure 10A). Specifically, treatment with pasireotide-LAR in parous mice increased the protective effect observed in the pregnancy and lactation group (P=0.0018) (Figure 10B). Moreover, the protective effect produced by pasireotide in nulliparous mice was equivalent to the one observed in parous mice with breastfeeding in terms of tumor latency; indeed, there were no statistical differences between both groups (Figure 10C). As expected, the protection was more evident compared with the nulliparous group (P<0.0001) (Figure 10D).

A protective effect of pasireotide-LAR treatment was also observed concerning the incidence of breast cancer (P<0.0001) (Figure 11A). The group of multiparous mice treated with pasireotide showed a lower tumor incidence (7 mice of 35) than the group of multiparous without treatment (19 mice of 32) (P = 0.001) (Figure 11B). Although the treated nulliparous mice equaled the untreated multiparous ones in terms of tumor latency (Figure 10C), no differences in tumor incidence were observed (Figure 11C). As expected, there was a significant difference in tumor incidence between extreme susceptibility groups, untreated nulliparous (31 out of 35 mice developed tumor), and multiparous mice treated with pasireotide (7 out of 35 mice developed tumor) (P<0.0001) (Figure 11D).

Finally, we also observed a decrease in tumor multiplicity (and in the number of tumors), understood as the percentage of mice with cancer that developed two or more mammary tumors. Global differences in multiplicity were observed (p<0.0001) (Figure 11E), with no differences between treated and untreated multiparous mice with pasireotide (Figure 11F). Although pasireotide induced in nulliparous mice protection, in terms of latency, similar to the multiparous mice without treatment (Figure 5C) and there were no differences in incidence (Figure 11C), however, the multiplicity was lower in untreated multiparous mice than in the treated nulliparous ones (Figure 11G). The significant differences between extreme risk groups (treated multiparous versus untreated nulliparous) were evident (Figure 11H).

In conclusion, pasireotide potentiates the protective effect of pregnancy in the Brca1/P53 deficient model in terms of latency and tumor incidence.

### Example 12. Evaluation of tumor evolution in Brca1/P53 deficient multiparous mice treated with pasireotide

Subsequently, we evaluated the evolution of breast cancer treated with pasireotide, continuing with the same dose used in chemoprevention. Regarding temporal parameters of the disease, no differences were observed in the duration of the disease. Also, no differences were observed in overall survival (Figure 12A, Figure 12B). A lower tumor growth rate was observed (p= 0.0209) (Figure 12C). Regarding long-distance dissemination, differences were observed in the incidence of metastases (P=0.0375) and multiplicity (P=0.0375). Of note, no metastases were observed in any of the pasireotide-treated mice. This result is remarkable, considering that these mice also develop skin tumors (Figure 12D, Figure 12E). Thus pasireotide could have value in secondary chemoprevention and low the risk of breast cancer dissemination

### Example 13. THESIS Evaluation of the evolution of the disease at the histopathological level in multiparous mice treated with pasireotide

After necropsy of the animal and once the tumors were removed, the existence of possible changes in the histopathological behavior of the disease between multiparous mice treated and untreated with pasireotide was studied. As detailed in section 1.2 of this results section, 76.4% of the tumors found in multiparous mice were diagnosed as infiltrating ductal carcinoma. Regarding the degree of differentiation, 41.2% of the tumors were classified as well or moderately differentiated. However, in multiparous mice receiving somatostatin analog treatment, 100% of tumors were diagnosed as infiltrating ductal carcinoma. Despite these percentage differences, no statistically significant differences were observed (P = 0.2688) (Figure 13E). Regarding the degree of differentiation, 100% of the tumors of the mice in the pasireotide treatment regimen were well differentiated, showing statistically significant differences with the multiparous ones that had not received treatment (P = 0.0170) (Figure 13F).

Thus, pasireotide treatment was associated with less aggressive histopathological disease, with a predominance of well- and moderately-differentiated tumors over lesser-differentiated ones in untreated mice.

### Example 14. Evaluation of the proliferation (Ki67) in the epithelial cells of the mammary gland at five months after treatment with pasireotide

Our working hypothesis is that pasireotide could enhance the antiproliferative effect of pregnancy in the epithelial component of the mammary gland. Thus, we evaluated the pasireotide effect in proliferation. *Brca1*/*P53* deficient mice were evaluated at five months of age, nulliparous and multiparous (coinciding with 60 days after weaning.) So, Ki67-positive cells detected by immunohistochemistry were quantified by Leica Application Suite software.

An ostensible decrease in basal epithelial cell proliferation was observed in nulliparous mice treated with pasireotide compared to untreated ones (Figure 14A). The same was observed for pasireotide-treated multiparous mice relative to untreated controls (Figure 14B).

### Example 15. Evaluation of the ductal wall area of the mammary gland at five months after treatment with pasireotide

Since the mice treated with pasireotide had a lower basal proliferation of the epithelial component of the mammary gland, we then evaluated whether this reduced proliferation could have had an impact on the ductal wall component.

Thus, the same mice evaluated for proliferation were now evaluated for ductal wall area by ImageJ software. A statistically significant decrease in the ductal component was observed between pasireotide-treated versus untreated nulliparous Brca1/P53-deficient mice (P=0.0055) (Figure 15A). Similarly, a dramatic decrease in the ductal component was observed in pasireotide-treated multiparous Brca1/P53-deficient mice (P=0.0062) (Figure 15B).

### Example 16. Comparison of epithelial proliferation between multiparous mice treated and untreated with pasireotide

After observing that pregnancy produced a reduction in basal epithelial proliferation, the effect of pasireotide on epithelial proliferation itself was evaluated in multiparous mice deficient in Brca1/P53. It was observed that multiparous mice that received treatment with the somatostatin analog had less epithelial proliferation than multiparous mice that did not receive treatment (P < 0.0001) (Figure 16A). It was found that this phenomenon also occurred both in animals with low tumor latency (P < 0.0001) (Figure 16B), and with high tumor latency (P < 0.0001) (Figure 16C).

### Example 17. Evaluation of the association between epithelial proliferation and intragroup tumor latency in multiparous mice treated and untreated with pasireotide

Subsequently, the relationship between breast epithelial proliferation and breast cancer latency was studied in pasireotide-treated and untreated multiparous mice. As already described, in multiparous mice that did not receive treatment, no statistically significant differences in epithelial proliferation were observed between mice with low and high tumor latency (P = 0.8461). Furthermore, no correlation was found between epithelial proliferation and tumor latency (P = 0.7003, r = -0.0590).

When the group of multiparous mice that received treatment with the somatostatin analog was studied, a trend was observed, although not statistically significant, towards an increase in epithelial proliferation in mice with high tumor latency, compared to those with low latency (P = 0.0610) (Figure 17A). However, as seen in the previous section, it should be remembered that, compared to untreated multiparous mice, pasireotide induced a potent hypoproliferative state of the ductal epithelium (Figure 16 A-C). No correlation was found between proliferation and tumor latency (P = 0.1379, r = 0.2273) (Figure 37B), nor between it and survival.

In summary, it was evaluated in the previous section how pasireotide produced a basal hypoproliferative state in the mammary epithelium of multiparous mice. In this section, it was found that this hypoproliferative state remained unchanged over time.

### Example 18. Comparison of epithelial proliferation between nulliparous mice treated and untreated with pasireotide

The effect of pasireotide on the proliferation of the mammary epithelium of nulliparous mice deficient in Brca1 and P53 was studied. A highly statistically significant decrease in epithelial proliferation was observed in treated versus untreated mice (P < 0.0001) (Figure 18A). As in the multiparous mice, the animals that received pasireotide treatment showed a lower proliferative index than their untreated counterparts, both in mice with low tumor latency (P < 0.0001) (Figure 18B) and in those with high latency (P < 0.0001) (Figure 18C).

### Example 19. Evaluation of the relationship between epithelial proliferation and intragroup tumor latency in nulliparous mice treated and untreated with pasireotide

Subsequently, the relationship between breast epithelial proliferation and breast cancer latency in pasireotide-treated and untreated Brca1/P53-deficient nulliparous mice was studied. In nulliparous mice not treated as described above, no statistically significant differences in proliferation were observed between mice with high and low tumor latency. There was also no correlation between proliferation and latency.

However, in treated gilts, mice with low tumor latency had significantly greater epithelial proliferation than those with high latency (P = 0.0099) (Figure 19A). Similarly, a negative correlation was also observed between epithelial proliferation and tumor latency (P = 0.0003, r = -0.4915), such that mice with higher latency showed lower proliferation and vice versa (Figure 19B). The same negative correlation was observed between epithelial proliferation and survival.

In summary, pasireotide treatment in Brca1/P53-deficient nulliparous mice induced a hypoproliferative state of the ductal epithelium. Furthermore, epithelial proliferation was less and less as the age of the mice progressed.

### Example 20. Effect of pasireotide treatment on the proportion of the P53 allele deleted

Subsequently, the effect of the administration of the somatostatin analog was evaluated in mice deficient in Brca1/P53, on the proportion of the deleted or recombined allele of P53, both in nulliparous and multiparous mice.

### Example 21. Evaluation of the proportion of P53 allele deleted in multiparous Brca1/P53 knockout mice treated with pasireotide

After administration of pasireotide to multiparous Brca1/P53 deficient mice, they were observed to have a lower proportion of recombined allele of P53 than those multiparous that were not treated (P = 0.0226) (Figure 20A). Furthermore, no correlation was observed between the proportion of recombined P53 allele and tumor latency (P = 0.9659, r = -0.0167) (Figure 20B), nor with survival.

This result indicates a specific decrease in the proportion of the recombined allele of P53 and, indirectly, of recombinant cells in the treated multiparous compared to the untreated ones.

### Example 22. Assessment of Recombinant P53 Allele Proportion in Pasireotide-Treated Nulliparous Brca1/P53 Deficient Mice

In the same way, the proportion of the recombined P53 allele was evaluated in nulliparous mice that received treatment with the somatostatin analog.

In these mice no statistically significant differences were observed for the fraction of P53 allele recombined in the breast (P = 0.7633) (Figure 21A). There was also no correlation between the RQ of the P53 recombinant allele and tumor latency (P = 0.9434, r = -0.0209) (Figure 21B), nor with survival.

### Example 23. Quantification of the ductal epithelial area of the breasts

When comparing the ductal epithelial area of nulliparous with that of their multiparous counterparts in 160-day-old mice that did not receive treatment, no statistically significant differences were observed (P = 0.6071) (Figure 22A). In contrast, when comparing nulliparous mice treated and untreated with pasireotide, it was observed that those who received treatment showed a lower percentage of ductal epithelial area, which was statistically highly significant (P < 0.0001); in fact, the median epithelial area in the untreated group was 8.87% and in the pasireotide group it was 0.70% (Figure 22B).

The same result was observed when comparing the ductal epithelial area of multiparous mice with and without treatment. The untreated animals showed a median ductal area of 9.49%, while in the treated it was 1.45% (P < 0.0001) (Figure 22C).

Finally, when both treated groups, nulliparous versus multiparous, were compared, it was observed how non-pregnant mice showed a lower percentage of the ductal epithelial component (0.70%) compared to multiparous (1.45%), with a statistically significant difference (P= 0.0089) (Figure 22D). This result can perhaps be attributed to a higher ductal residue after pregnancy and lactation and that postlactational involution could not eliminate.

Therefore, at 160 days of age we found no differences in the epithelial area between nulliparous and multiparous. However, pasireotide treatment dramatically decreased ductal area in both groups relative to their untreated counterparts, and thus is an age-independent effect. Finally, the treated multiparous mice showed somewhat more epithelial area than the treated nulliparous mice, but the lower tumor susceptibility of these treated multiparous mice with respect to their treated nulliparous counterparts indicates qualitative changes in the behavior of the epithelium that make it less susceptible to malignancy.

### Example 24. Histopathological evaluation of the breasts

Histopathological evaluation of the breasts was then carried out to determine if the treatment induced changes in the tissue related to greater or lesser tumor susceptibility. The tissues were evaluated by an expert in Pathological Anatomy from the Comparative Molecular Pathology Service of the Cancer Research Center (Figure 23).

Histopathologically, in untreated nulliparous mice, the ducts were small and isolated, formed by a simple cuboidal epithelium, although they often showed signs of atypical hyperplasia with the accumulation of abnormal cells in the wall of the mammary ducts. Therefore, although in many cases the structure of the ducts remained preserved, it was not uncommon to find these atypical hyperplasias with ducts with rows of cells arranged in disorderly accumulations that sometimes came to occupy the lumen of the duct. Cellular morphology showed evident nucleoli. Regarding the adipocytic component, mature fat prevailed over immature fat in a 70/30 ratio (Figure 23A).

The multiparous without treatment showed a smaller number of ducts and larger ones. These ducts were isolated and without dilations, although some contained proteinaceous material. The ductal morphology was normal, with the majority of the ducts preserved, with some unremarkable typical hyperplasia. The adipocyte component was predominantly formed by immature fat of multivacuolated cells, of vacuole of intermediate size, in a proportion of 60/40 with respect to mature fat (Figure 23B). Nulliparous females receiving pasireotide treatment showed sparse, isolated ducts with normal lining cells, undilated ducts, and were smaller in size than those of the previous groups. In practically all the ducts, hyperplasias were not observed and in those rare cases where they were found, they were typical hyperplasias, without alterations in cell morphology, so no signs of malignancy were suspected. The adipose tissue was predominantly mature fat, although some foci of immature fat were sometimes observed (Figure 23C).

Finally, in multiparous mice treated with pasireotide, it was observed that the breasts contained a very small number of ducts, formed mostly by few cells, they were isolated and showed no signs of cystic dilatation. The ducts were formed by normal lining epithelium; in some cases proteinaceous content was observed in the lumen, but it was not common. The adipocytic component, for the most part, was made up of mature fat, although foci of immature fat from vacuole intermedia could be observed at both ends of the breast (Figure 23D).

In summary, ductal epithelium with some signs of malignancy was more present in untreated nulliparous. Pregnancy decreased the number of ducts and the epithelium had a more benign appearance than that of nulliparous. It was striking that immature adipose tissue predominated, which is interpreted as a result of its expansion after pregnancy. Pasireotide treatment in nulliparous mice decreased the number of ducts, and their epithelium had a more benign appearance than that of nulliparous mice without treatment. Treatment with pasireotide seems to have limited this expansion of immature adipose tissue, given that mature adipose tissue predominated in treated multiparous mice.

### Example 25. Assessment of ductal epithelial proliferation

Subsequently, the proliferation of the ductal epithelium was evaluated by immunohistochemistry against Ki67. Thus, statistically significant differences were observed between the nulliparous and multiparous groups (P = 0.0019), with the nulliparous group having the highest number of Ki67 positive cells (22.64%), compared to the multiparous group, which showed less proliferation (12.33%). (Figure 24A).

When proliferation was compared between treated and untreated nulliparous mice, it was observed that those nulliparous mice that received the drug showed a lower proliferative index (2.43%) than those that did not receive it (22.64%), with statistically significant differences (P < 0.0001) (Figure 24B). In the case of multiparous mice, the same phenomenon was observed. Those mice that were treated with pasireotide showed significantly fewer Ki67 positive cells (1.50%) than untreated animals (12.33%) (P < 0.0001) (Figure 24C).

When comparing both groups of mice with treatment, the multiparous mice showed a significantly lower percentage of Ki67 positive cells (1.50%) compared to the nulliparous group (2.43%) (P = 0.0029) (Figure 24D).

Consequently, after pregnancy there was a decrease in the proliferation of the ductal epithelium. Pasireotide treatment also decreased ductal epithelial proliferation in nulliparous mice and also potentiated the antiproliferative effect of pregnancy.

### Example 26. Quantification of the ductal component in the longitudinal cohort in nulliparous Brca1/P53 deficient mice treated and untreated with pasireotide

We then evaluated whether there is an association between de ductal wall area and the susceptibility to breast cancer. Thus we quantified the ductal area with ImageJ software in mice deficient in Brca1/P53 that developed breast cancer (the longitudinal cohort). The aim here is to verify to what extent, at the intra-cohort level, the ductal area was associated with tumor latency.

As in mice at five months (transversal cohort), the ductal area was smaller in the mammary glands of pasireotide-treated than untreated mice of the longitudinal cohort (P=0.0086) (Figure 25A). Furthermore, within the high tumor latency mice, the difference in ductal area between the treated and untreated groups was highly significant (P= 0.0001) (Figure 25B); while when comparing the low latency mice of both groups, treated and untreated, no significant differences were observed (Figure 25C). These results suggest that when pasireotide was less effective in reducing the ductal area, tumor latency was not affected since they all have low latency (and it was the same in treated and untreated mice).

Furthermore, within the pasireotide-treated mice, those that developed the tumor late (with high latency) had significantly less ductal area than those that developed the tumor early (with short latency) (P=0.0001) (Figure 25E). However, in mice not treated with pasireotide, significant differences were observed between those who developed the tumor early and late (Figure 25D). These data agree that a negative correlation between ductal area and tumor latency was observed in the treated group (Figure 10G) but positively in the untreated group (Figure 25F). All this data suggest that treatment with pasireotide decreased the ductal area and increased tumor latency.

### Example 27. Quantification of the ductal component in the longitudinal cohort in multiparous pasireotide-treated Brca1/P53 deficient mice treated and untreated with pasireotide

As expected, the ductal area was reduced in the mammary glands of pasireotide-treated than untreated mice of the multiparous longitudinal cohort (P<0.0001) (Figure 26A). Moreover, within both high tumor latency mice (P<0.0001) and shorter tumor latency (P=0.0007), the difference in ductal area between the treated and untreated groups was also highly significant (P= 0.0001) (Figure 26B, Figure 26C).

Also, as in nulliparous mice, we observed similar results in multiparous mice. Indeed, within the pasireotide-treated mice, those that developed the tumor late (with high latency) had significantly less ductal area than those that developed the tumor early (with short latency) (P=0.0007) (Figure 26E). However, significant differences were not observed between those who developed the tumor with early and late latency (Figure 26D). These data agree that a negative correlation between ductal area and tumor latency was observed in the treated group (Figure 26G) but with an absence of correlation in the untreated group (Figure 26F). All this data suggest that treatment with pasireotide decreased the ductal area and increased tumor latency.

### Example 28. Quantification of the ductal component of the non-tumoral mammary glands in the longitudinal cohort in nulliparous ErbB2/Neu+ mice

Since treatment with pasireotide also decreased the risk of breast cancer in the *ErbB2*/*Neu+* mice, we evaluated the ductal area in the non-tumoral mammary glands of mice that developed breast tumors with short and long latency. Thus, the ductal component was quantified using free software (Image J) in mice that developed tumors with long and short tumor latency. So, five mice with low tumor latency and 5 with high tumor latency were compared. Nulliparous ErbB2/Neu+ mice without treatment were included (Figure 27).

Mice treated with pasireotide had significantly less ductal area than untreated mice (P=0.001) (Figure 27A). In the control group, no correlation was observed between ductal area and tumor latency, and therefore, there were no differences between mice with high and low tumor latency (Figure 27B, Figure 27C).

However, significant differences were observed in nulliparous *Erbb2*/*Neu+* mice treated with pasireotide. So mice that developed tumor early (short latency) had significantly more ductal area (P=0.001) and a negative correlation between latency and ductal area (P=0.001; R= -0.4702) (Figure 27D, Figure 27E).

### Example 29. Quantification of ductal proliferation in the longitudinal cohort in nulliparous ErbB2/Neu+ mice: Less epithelial proliferation is associated with lower tumor susceptibility.

Subsequently, we evaluated whether the decreased ductal component was associated with decreased proliferation in the non-tumorous mammary glands of *ErbB2*/*Neu+* nulliparous mice. To this end, proliferation was evaluated in mice treated with pasireotide versus controls by detecting Ki-67 expression by immunohistochemistry and quantifying with the Leica Application Suite software. Mice treated with pasireotide had significantly less proliferation than untreated mice (P=0.001) (Figure 28A). In the control group, no correlation was observed between ductal area and tumor latency, and therefore, there were no differences between mice with high and low tumor latency (Figure 28B, Figure 28C).

Although in the *ErbB2*/*Neu+* controls, no differences were observed in the ductal area between mice with high and low latency (Figure 28B, Figure 28C), significant differences were observed in the degree of basal proliferation. Mice that developed tumors early (with low tumor latency) had non-tumorous mammary glands with higher basal proliferation (P=0.001) and a negative correlation between proliferation and tumor latency (P=0.001 and R=-0.6896) (Figure 28B, Figure 28C).

In the group of treated *EraB2*/Neu+ mice, the same was observed as in the controls. Mice that early developed tumors (short tumor latency) showed higher basal proliferation in non-tumorous mammary glands (P=0.001) and a negative correlation between proliferation and tumor latency (P=0.001 and R=-0.8159) (Figure 13D, E).

### Example 30. Quantification of recombinant epithelial cells by flow cytometry

We resorted to a direct detection method to evaluate differences between groups in the proportion of recombinant cells that lost P53 and Brca1. Thus, the proportion of recombinant cells was evaluated directly by detecting emitted fluorescence. To do this, mice deficient in *Brca1*/*P53,* carriers of Cre-recombinase under the promoter of cytokeratin 14, were crossed with mice carrying the red fluorescent protein (RFP) under the ROSA26 promoter. However, in the model, the RFP cDNA is preceded by a stop codon, flanked by LoxP sites, which prevents transcription of the fluorescent protein. After several crosses, mice deficient in *Brca1*/*P53* in the mammary glands were generated, in whose recombinant cells the Cre recombinase inactivates *Brca1* and *P53* and eliminates the stop codon and the recombinant cells emit red fluorescence. We carried out flow cytometry to quantify RFP+ cells (Figure 29A). Nulliparous and multiparous lactating mice, treated and untreated with pasireotide, were evaluated.

### Example 31. Evaluation of RFP+ cells in nulliparous mice

We first evaluated nulliparous mice. In quantifying RFP+ Lin- recombinant cells, no significant differences were observed in the proportion of recombinant cells between groups at five months between treated and untreated mice, in both wild type (WT) and Brca1/P53-deficient mice (Figure 29B). In order not to identify epithelial cells only by exclusion (i.e., non-stromal cells: Lin+, RFP+), the percentage of EpCAM+ RFP+ cells (EpCAM is a pan-epithelial marker) within epithelial cells (Lin-, RFP+) was also estimated.

Interestingly, the WT mice presented an expansion of basal cells after treatment with pasireotide that was not present in the recombinant cells of the Brca1/P53-deficient mice (Figure 29C).

The data observed at five months by FACS show no significant differences between treated and untreated *Brca1*/*P53*-deficient mice in the percentage of recombinant cells. It does not mean that there is no decrease in the absolute number of recombinant cells. Just the opposite, as we have seen that at 60 days, there is a significant reduction in the ductal epithelial component (Figure 14).

### Example 32. Evaluation of RFP+ cells in multiparous mice

We have obtained similar results in multiparous Brca1/P53-deficient mice (Figure 30).

### Example 33. Assessment of the ratio of the luminal and basal components in nulliparous Brca1/P53-deficient mice

Given that the tumor is derived from the recombinant cells, which lose *Brca1* and *P53,* and are marked with red fluorescence in this model, we evaluated whether there were quantitative changes in subpopulations within the recombinant (RFP+) cells between the study groups at five months.

In the mammary gland, there are two significant subgroups of epithelial cells, those that are towards the lumen of the tubules or luminal cells, and those outsides of them, next to the basal membrane, which are the basal cells and are myoepithelial. These two subpopulations can be distinguished (i) by the degree of expression of the pan-epithelial marker EpCAM, which is higher in luminal cells, and (ii) of the CD49f marker which is more expressed in basal cells. Thus, luminal cells can be defined as EpCAM high (hi)/CD49f low (lo), and basal cells as EpCAM Io/CD49f hi.

No changes in the proportions of luminal or basal subpopulations were observed between treated and untreated Brca1/P53-deficient mice. However, in the WT mice, a discrete expansion of the basal component was observed and simultaneously a slight contraction of the luminal component (Figure 31A, Figure 31B). By referring to a larger cell population that includes the above cells (the Lin-) and prevents the basal/luminal ratio from being "communicating vessels," it was confirmed that in the treated WT mice, there is an expansion of the basal population that is not present in the treated KO mice (Figure 31C, Figure 31D).

### Example 34. Assessment of the ratio of the luminal and basal components in multiparous Brca1/P53-deficient mice

Regarding the two subpopulations of recombinant epithelial cells (RFP+), an increase in the proportion of luminal cells and the consequent decrease in the proportion of the complementary subpopulation, the basal cells, is observed. Interestingly, these changes were not observed in WT mice; it seems specific to those deficient in Brca1/P53 (Figure 32A, Figure 32B).

To assess which changes persist, we evaluated the proportion of recombinant luminal and basal cells, concerning a larger compartment, that of total epithelial cells (Lin-), and the same behavior was maintained: expansion of the luminal population and decrease of the basal one (Figure 32C, Figure 32D). These results suggest a specific effect of pasireotide on both subpopulations of epithelial cells that lose Brca1/P53.

Nonetheless, despite these qualitative changes at this scale, it is essential to remember that given the massive decrease in epithelium seen on immunohistochemistry, there will be a massive decrease in absolute terms in all epithelial subpopulations in pasireotide-treated mice (Figure 14).

### Example 35. Evaluation of the proportion of luminal cell subpopulations in nulliparous mice

Within the luminal cells, there are two main subpopulations, the ductal (including mature ductal and progenitor cells) and alveolar (including early progenitor, late progenitor, and mature alveolar cells). The formerly generated ducts conduct the milk produced by the alveolar cells during lactation, mainly the mature alveolar cells. During post-lactational involution, many of the luminal component generated during pregnancy and lactation is lost, both the ductal cells (arborizations) and the alveolar cells, especially the latter. After the involution, the arborizations are reduced, leaving fewer ducts; and mature milk-producing alveolar cells are also removed, leaving only early alveolar precursors. Subsequently, we evaluated whether the decrease in the luminal component after pregnancy equally affected ductal (Sca1+) and alveolar (Sca1-) cells or whether there were differences between the groups studied. Therefore, we determined whether there were differences in the proportion of Sca1 + cells within the subpopulation of total luminal cells (EpCAM hi/CD49f lo) recombinant (RFP+) (Figure 33A, Figure 33B). A decrease in Sca1+ ductal cells was observed concerning total RFP+ luminal cells in treated versus untreated mice in WT and Brca1/P53-deficient mice (Figure 33A). This result, as expected, was accompanied by the subsequent increase in the alveolar population (Figure 33B).

In addition, we cannot forget that the changes observed in ductal and alveolar cells concerning the total number of luminal cells are relative since both populations add up to the total number of luminal cells. When the changes observed for the total number of recombinant epithelial cells (RFP+) were considered, the decrease in luminal ductal cells was maintained (Figure 33C). However, the increase in alveolar luminal cells was no longer observed; there were no significant differences between groups (Figure 33D).

The reduction of the luminal component within recombinant cells is of importance, as *BRCA1*-deficient breast cancer has been shown to originate from luminal precursors. Therefore, the decrease in the luminal component within the recombinant cells suggests that pasireotide decreases the number of potential cellular targets that are the origin of the tumor. In absolute terms, this decrease would be even more significant after treatment because of the global reduction of the epithelial component (Figure 14).

### Example 36. Evaluation of the proportion of luminal cell subpopulations in multiparous mice

Subsequently, we evaluated the proportion of luminal subpopulations in the multiparous mice. A decrease in Sca1+ ductal precursors and a proportional decrease in the complementary subpopulation of Sca1- mature ductal cells were observed in both WT and Brca1/P53-deficient mice (Figure 34A, Figure 34B).

Next, we evaluated the luminal subpopulations against a more extensive, non-complementary compartment of total epithelial cells. In this case, only the alveolar cell subpopulation remains elevated, specifically in the Brca1/P53 knock-out mice (Figure 34C, Figure 34D). However, as repeated previously, in absolute terms, all epithelial compartments are decreased in mice treated with pasireotide, given its antiproliferative effect (Figure 14).

### Example 37. Evaluation of the proportion of ductal subpopulations in nulliparous mice

We then delved into the differences within the ductal population, specifically, between the proportions of progenitor (CD61 +) and mature (CD61-) luminal ductal cells. No differences were observed between nulliparous treated and untreated mice in WT and *Brca1*/*P53*-deficient mice.

No significant changes were observed in the percentage of mature ductal and ductal precursors in treated and untreated mice deficient in *Brca1*/*P53* and WT (Figure 35A, Figure 35B). When we compare them for the total epithelial cells, it was observed that the decrease in ductal cells affects the two subpopulations equally (Figure 35C, Figure 35D).

### Example 38. Evaluation of the proportion of ductal subpopulations in multiparous mice

Following on, the ratios of recombinant ductal (CD61+) and mature ductal (CD61-) precursors were evaluated in multiparous WT and *Brca1*/*P53*-deficient mice, and no significant differences were observed (Figure 36A, Figure 36B).

We then compared the proportions of ductal cells to the broader compartment of luminal cells. Interestingly, a specific decrease in the proportion of ductal precursor cells was observed (Figure 21B, C). The subpopulation that generates the tumor has been described, and we must insist on the concept that all epithelial subpopulations in absolute terms are decreased due to the antiproliferative effect of pasireotide (Figure 14).

### Example 39. Evaluation of luminal and basal epithelial subpopulations

In the mammary gland there are two main subgroups of epithelial cells, those that are close to the lumen of the ducts, or luminal; and those external to the lumen, close to the basement membrane, the basal cells. These two subpopulations can be distinguished by the degree of expression of the EpCAM marker, which is higher in luminal cells, and by the CD49f marker, which is higher in basal cells. Therefore, epithelial cells can be defined by their high EpCAM and low CD49f marking and basal cells by being low EpCAM and high CD49f. With this criterion, the quantitative changes in these two subpopulations were evaluated. Examples of the distribution of these two subpopulations are shown, in the flow cytometry analysis, in the different groups studied in the cross-sectional cohort (Figure 37A).

### Example 40. Assessment of Recombinant Basal and Luminal Subpopulations

Since the breast tumor derives from the recombinant cells that have lost Brca1 and P53, and these are labeled with red fluorescence in this mouse model, the quantitative changes in these two RFP+ recombinant subpopulations with respect to the total of Lin- cells were studied. , between the different study groups of the cross-sectional cohort.

First, the effect that pregnancy and lactation had on these two recombinant cell subpopulations was studied, for which multiparous and nulliparous were compared. Thus, a statistically significant decrease in the luminal population was observed in multiparous mice compared to nulliparous mice (P = 0.0227) (Figure 37B). However, no differences were observed in the baseline population (P = 0.7040) (Figure 37C).

Afterwards, the effect of pasireotide was studied in the nulliparous group, for which treated and untreated animals were compared. No differences were observed between both groups in the proportion of luminal cells (P = 0.2660) (Figure 37D). There were also no differences in the baseline population (P = 1,000) (Figure 37E).

Finally, the effect of pasireotide treatment in multiparous mice was studied; and treated and untreated multiparous were compared. A statistically significant increase in luminal population was observed in treated multiparous (P = 0.0185) (Figure 37F). When studying the basal cell population, a statistically suggestive reduction was observed in treated multiparous compared to control multiparous (P = 0.0774) (Figure 37G).

In summary, pregnancy produced a decrease in the proportion of luminal cells, without modification of the basal cells. Pasireotide treatment did not significantly change the proportion of these two subpopulations in gilts. However, the apparently paradoxical increase in the luminal subpopulation in treated multiparous mice compared to untreated mice was striking.

### Example 41. Evaluation of non-recombinant luminal and basal subpopulations

To determine if the observed changes were directly associated with the recombined *Brca1*/*P53* component, or if, on the contrary, they were also present in these same luminal and basal subpopulations, but in non-recombinant cells, the study was repeated in RFP-negative cells (RFP-). with respect to the total number of negative lineage cells.

When the effect of pregnancy on non-recombinant cells was studied, an effect similar to that exhibited by recombinant RFP+ cells was observed. Thus, a statistically significant reduction in the luminal RFP-population was observed in multiparous relative to nulliparous mice (P = 0.0482) (Figure 38A, left), with no change in the basal subpopulation (P = 0.5433) (Figure 38A, right)).

Regarding the effect of pasireotide in nulliparous mice on non-recombinant cells; he also observed the same as with the recombinants. That is, no differences were observed with their untreated counterparts, neither in the luminal population (P = 0.1384) (Figure 38B, left), nor in the basal population (P = 0.3408) (Figure 38B, right).

Finally, when comparing the treated and untreated multiparous groups, a statistically significant increase in the luminal subpopulation (P = 0.0372) was observed, as in the recombinant cells (Figure 39C, left), with no differences in the basal (P = 0.2212) (Figure 38C, right).

In summary, pregnancy and pasireotide induced the same changes in the proportions of the luminal subpopulation, with no changes in the basal, in both recombinant RFP+ and non-recombinant RFP-cells.

### Example 42. Evaluation of ductal and alveolar luminal subpopulations

The luminal population is made up of two cell subpopulations, the ductal (which includes ductal progenitor cells and mature ductal cells) and the alveolar (which includes early alveolar progenitor cells, late progenitor cells, and mature alveolar cells). During lactation, the ducts generated during pregnancy carry the milk produced by the alveolar cells. Subsequently, during post-lactation involution, a large part of the luminal component is lost, both of ductal and alveolar cells, all these subpopulations being reduced. Ductal cells express the Sca1 marker, while alveolar cells are Sca1 negative. The distribution of these cell subpopulations, within the different groups of mice in the cross-sectional cohort, is shown in Figure 39A.

### Example 43. Evaluation of Recombinant Ductal and Alveolar Luminal Subpopulations

First, the effect of pregnancy on ductal and alveolar populations with respect to total recombinant EpCAM+ cells was studied. A statistically significant decrease in Sca1+ ductal population was observed in multiparous relative to nulliparous mice (P = 0.0007) (Figure 39B); meanwhile, no differences were observed in the alveolar subpopulation Sca1- (P = 0.1965) (Figure 39C).

The effect of pasireotide on these same cell subpopulations in nulliparous mice was then evaluated. A statistically significant decrease in the ductal Sca1+ subpopulation was observed in nulliparous mice treated with pasireotide (P = 0.0111) compared to untreated mice (Figure 39D). No differences in alveolar Sca1- subpopulation were observed between treated and untreated gilts (P = 0.5966) (Figure 39E).

Finally, the effect of pasireotide in multiparous mice was evaluated. No differences were observed in the Sca1+ ductal population (P = 0.7859) (Figure 39F). However, an increase in the alveolar Sca1-population was observed in treated multiparous compared to untreated ones (P = 0.0011) (Figure 39G).

In summary, pregnancy and treatment with pasireotide in nulliparous mice produce a decrease in the proportion of ductal cells, without modification of the alveolar cells, with respect to the total number of EpCAM+ recombinant cells. In multiparous mice treated with pasireotide, we did not observe differences in the proportion of ductal cells, however, an increase in the proportion of alveolar cells was observed.

### Example 44. Evaluation of non-recombinant ductal and alveolar luminal subpopulations

Then, as in previous analyses, it was evaluated whether this effect was specific to recombinant cells or whether it was also present in the same subpopulations of non-recombinant cells. Therefore, the proportions of these subpopulations in the non-recombinant RFP- cells were evaluated.

Regarding the effect of pregnancy on these subpopulations, a decrease in the ductal Sca1+ subpopulation was observed in multiparous versus nulliparous mice (P = 0.0014) (Figure 40A, left). No differences in alveolar Sca1- population were observed between nulliparous and multiparous mice (P = 0.3233) (Figure 40A, right). Therefore, the effect of pregnancy is the same on these subpopulations, ductal and alveolar, both in recombinant (RFP+) and non-recombinant (RFP-) cells. The effect of the drug on these two luminal subpopulations was then studied. In treated nulliparous, as in the RFP+ cells shown in the previous section, a statistically significant decrease in the Sca1+ ductal population was observed compared to untreated ones (P = 0.0111) (Figure 40B, left). However, a slight but significant increase in alveolar Sca1- cells was also observed relative to the control group (P = 0.0443) (Figure 40B, right), which was not present in the recombinant RFP+ cells.

In the multiparous group, no statistically significant differences were observed between treated and untreated in the Sca1+ ductal population (P = 0.4150) (Figure 40C, left). A statistically significant increase in the Sca1- population was observed in the group of multiparous mice treated with pasireotide compared to those not treated (P = 0.0057) (Figure 40C, right). Both effects were also observed in the recombinant RFP+ subpopulations.

In summary, the same changes in the proportion of ductal and alveolar cells in RFP-negative cells were observed, with the exception of a modest increase in alveolar cells in the pasireotide-treated gilt group.

### Example 45. Evaluation of recombinant ductal luminal subpopulations (progenitor and mature)

Next, the differences within the ductal population were studied, differentiating between ductal progenitors (CD61+) and mature ductal cells (CD61-), with respect to the total number of recombinant luminal cells (RFP+). First, the effect of pregnancy in these populations was analyzed. A statistically significant reduction in both the ductal progenitor subpopulation, CD61+ (P = 0.0098) (Figure 41A), and the mature ductal subpopulation, CD61- (P = 0.0204) (Figure 41B) were observed in multiparous mice compared to the nulliparous.

These subpopulations were then studied in the mice that received the somatostatin analog. In the group of treated gilts, with respect to the untreated ones, a statistically significant reduction was also observed in the progenitor subpopulations, CD61+ (P= 0.0022) (Figure 41C), and in the mature subpopulations, CD61- (P = 0.0059) (Figure 41D).

In the group of multiparous mice treated with pasireotide, a statistically significant decrease in the progenitor subpopulation, CD61+ (P = 0.0145) was observed (Figure 41E); however, no differences were observed in the CD61- subpopulation (P= 0.1743) (Figure 41F).

Consequently, pregnancy produced a decrease in the proportion of ductal cells, both mature and progenitor, with respect to the total luminal cells. This effect was also seen in pasireotide-treated gilts. In addition, the decrease in ductal progenitors was enhanced in treated multiparous compared to untreated ones.

### Example 46. Evaluation of non-recombinant ductal luminal subpopulations (progenitor and mature)

As in previous studies, later, to assess whether the effect produced by pasireotide and that of pregnancy were specific or not to the recombinant luminal population, these same ductal subpopulations were analyzed in RFP- cells, which do not recombine *Brca1*/*P53.* When analyzing the effect of pregnancy, the same behavior was observed as in RFP+, a reduction in ductal progenitors (P = 0.0334) (Figure 43A, left), and in mature ductal cells (P = 0.0024) (Figure 42A, right).

Next, the effect of pasireotide administration on nulliparous mice was studied. Again, as in the RFP+ population, there was a decrease in both ductal subpopulations, progenitors (P = 0.0010) (Figure 42B, left), and mature ductal cells (P = 0.0343) (Figure 42B, right).

Finally, the effect of pasireotide in multiparous mice was studied. A statistically significant decrease was observed in the ductal progenitor population (P=0.0086) (Figure 42C, left), but no statistically significant difference in the mature ductal population (p=0.1029) (Figure 42C, right).

Therefore, the reduction produced by pregnancy and pasireotide treatment on ductal subpopulations also affects non-recombinant cells.

### Example 47. Evaluation of differentially expressed genes between the different study groups

Next, the differences in gene expression that could justify the protective effect of pregnancy and treatment with pasireotide against breast cancer were studied. For this, RNA-Seq studies were performed on RNA extracted from the organoids of mice.

First, the number of genes expressed in each study group of the cross-sectional cohort was evaluated. To do this, two-by-two comparisons were made and the number of genes expressed in common to each pair of groups and specific to each of them was identified. Then, the number of differentially expressed genes (DEGs) was identified and whether these were overexpressed or underexpressed with respect to the reference group.

A total of 40,775 genes expressed in gilts and 38,623 in multiparous were observed, of which 30,973 were commonly expressed genes, 9,802 genes were specifically expressed in gilts, and 7,650 in multiparous (Figure 43A). Regarding the DEGs, it was observed that in multiparous mice there were 567 overexpressed genes and 1459 underexpressed with respect to the nulliparous group (Figure 43B).

Regarding gilts treated and untreated with pasireotide, it was observed that in treated gilts, of the 33,757 expressed, they shared 26,016 with their untreated counterparts and 7,741 genes were found to be specifically expressed in the former (Figure 43C). Of the genes commonly expressed between the two groups, 960 over-expressed and 2528 under-expressed DEGs were observed in treated gilts relative to untreated mice (Figure 43D).

In the case of pasireotide-treated multiparous mice, 36,775 expressed genes were observed, of which 27,118 were shared with untreated multiparous mice and 9,657 were specifically expressed in treated mice (Figure 43E). Of these 27,118 common genes, 657 DEGs were found to be overexpressed and 1,061 underexpressed relative to untreated gilts (Figure 43F).

### Example 48. Enrichment analysis of differentially expressed genes

Next, enrichment studies were performed to identify functional groups where these differentially expressed genes were integrated. From these Gene Ontology studies, the biological processes involved in different overexpressed and underexpressed functions were selected. Some of the most represented functions in each of the comparisons were catabolic and biosynthetic processes, or the metabolism of nitrogenous compounds.

Subsequently, given that in this work it has been observed that the decrease in proliferation is a key function associated with less susceptibility to breast cancer, both after pregnancy and after treatment with somatostatin analog, DEGs genes were delved into the different situations (comparisons) associated with processes related to proliferation.

In multiparous mice with respect to nulliparous mice, an underexpression of genes directly or indirectly related to proliferation was observed, such as proliferation per se, mRNA processing, chromosomal organization, cell cycle and mitosis, among others.

Regarding the effect of pasireotide in nulliparous mice with respect to their untreated counterparts, an overexpression was observed in cell death processes, as well as an underexpression in cell proliferation and in functions related to growth.

Finally, in multiparous mice treated with pasireotide, compared to untreated mice, an underexpression was observed in genes related to chromosomal organization, mRNA processing and the assembly of ribonucleoprotein complexes.

### Example 49. Identification of genes and main differential signaling pathways involved in proliferation pathways through enrichment studies

Then, the identification of the genes and main differential signaling pathways involved in the hypoproliferative state that has been observed to be associated with lower susceptibility to breast cancer was delved into.

When comparing multiparous mice with nulliparous mice, and delving into the pathways related to the protective effect of pregnancy, it was observed that in multiparous mice there was underexpression at the transcriptomic level of pathways specifically related to proliferation in the breast, such as pathways activated by estrogens and ErbB2, and overlapping with them, other essential pathways such as Pi3k-Akt, the Jak-Stat pathway, the Ras pathway, Wnt and Tgf-beta and pathways generically involved in cancer, among others.

Modified proliferation pathways were then identified after pasireotide treatment in the absence of pregnancy. Here, an underexpression of practically the same pathways was observed, such as in the MAPK pathway, in PI3K-Akt, in the estrogen signaling pathway, in ErbB2, in the TGF-β pathway or in the Ras pathway. This is very interesting because it suggests that pasireotide treatment reproduces, at least in part, the effect that pregnancy has on multiple signaling pathways.

Finally, the main pathways involved in proliferation that were underexpressed in treated multiparous mice with respect to their untreated counterparts were evaluated. It was observed how pasireotide was able to further underexpress some of the pathways that already decreased pregnancy, such as the PI3K-Akt pathway, pathways involved in cancer, the MAPK pathway or the estrogen and ErbB signaling pathway.

### Example 50. Evaluation of the effect of pasireotide in in vitro models

After the results obtained on the effects of pasireotide in in vivo mouse models, its effects in in vitro models were evaluated in order to delve into cellular and molecular mechanisms that would help explain the results obtained. For this, four cell lines (described in materials and methods) of human and mouse origin were used. To delve into cellular and molecular mechanisms related to tumor prevention, two non-tumor breast lines were chosen, MCF10A, obtained from non-tumor tissue from a premenopausal woman, and the HC11 line, derived from the normal mammary epithelium of a pregnant mouse. In order to delve into the mechanisms related to changes in tumor evolution, two breast cancer lines were selected, AU565, derived from a HER2/NEU positive tumor, and HCC1937, which have a triple-negative pattern with a BRCA1 mutation.

### Example 51. Evaluation of the effect of pasireotide on non-tumor breast cell lines

Prior to studies of the effect of pasireotide on the two non-tumor lines (HC11 and MCF10A), the expression of somatostatin receptors was evaluated by QPCR.

In the HC11 line, expression of all Sstr receptors for which pasireotide has affinity was observed, showing greater expression of Sstr5 (Figure 44A).

In the MCF10A cell line, expression of all somatostatin receptors was also observed, however, here SSTR1 was the most expressed (Figure 44B).

### Example 52. Viability study in the HC11 line without lactogenic differentiation and in the MCF10A line

After studying the expression of somatostatin receptors in non-tumor cell lines, the effect of pasireotide on cell viability, proliferation and death was studied.

Initially, the viability of cell lines treated at different concentrations of the drug (5 µM, 10 µM and 20 µM), for 48 and 72 hours, was evaluated using MTT assays.

In the HC11 line, at a concentration of 20 µM and after 48 hours of treatment, a reduction in viability of 66.5% was observed compared to untreated cells (Figure 45A).

The same assay was repeated in the MCF10A cell line, also observing a 70.4% reduction in viability in cells treated with pasireotide under the same conditions as above, at a concentration of 20 µM for 48 hours (Figure 45B).

This reduction in cell viability after pasireotide treatment was confirmed by phase contrast microscopy. Furthermore, in both cell lines, much slower growth was observed after treatment (Figure 46).

### Example 53. Comparison of viability in HC11 cells before and after lactogenic differentiation

The HC11 cell line has the capacity for lactogenic differentiation under a hormonal cocktail. This differentiation consists of the expression of some of the milk proteins, such as beta-casein and lactalbumin, and the appearance of vesicles in the cytoplasm. After withdrawal of the hormonal cocktail, many of the cells die by apoptosis. For this reason, it is a cellular model frequently used to study in vitro the process of lactogenic differentiation and subsequent post-lactation involution.

In the HC11 line, one can distinguish between (i) cells before lactogenic differentiation (undifferentiated or predifferentiated), (ii) differentiated cells (which are exposed to the differentiation cocktail), (iii) after removing the cocktail, a massive apoptosis in which most of the cells die and (iv) post-differentiated after having gone through the differentiation process and that did not die by apoptosis after removal of the differentiation cocktail. In this work, the effect of pasireotide on predifferentiated and postdifferentiated cells was compared.

First, as was done with undifferentiated cells, the expression of Sstr was evaluated by QPCR in the differentiated cell line. A large increase in the expression of the four Sstr evaluated was observed compared to the undifferentiated line (Figure 47A).

Once the expression of somatostatin receptors was verified, the basal viability of the cells was compared, without treatment, according to their state of differentiation by means of MTT assays. Thus, the post-differentiated cells showed a lower viability than the undifferentiated ones, with a reduction of 15% (Figure 47B).

Next, cell viability was studied after treatment with pasireotide in post-differentiated HC11 cells, at different concentrations of the drug (10 µM, 15 µM and 20 µM) and after 48 and 72 hours of exposure to it.

A 50% reduction in viability was observed in cells treated at a concentration of 20 µM pasireotide for 48 hours (Figure 47C).

### Example 54. Evaluation of the effect of pasireotide on cell proliferation

Once the decrease in cell viability was observed after treatment with pasireotide, it was studied whether this phenomenon was caused by a reduction in proliferation. To do this, it was determined with the BrdU assay to accurately quantify proliferating cells (in S phase) and, simultaneously, the proportion of cells in the rest of the cell cycle phases. The study was carried out in non-tumor cell lines, HC11 and MCF10A.

Thus, undifferentiated HC11 cells were treated with pasireotide (20 µM) for 48 hours. In cells receiving pasireotide treatment, a statistically significant decrease in S-phase cells was observed. In addition, there was an increase in the number of cells arrested in G0/G1 and G2/M phases, also statistically significant compared to untreated cells (Figure 48).

In MCF10A cells, due to their slow growth and, therefore, the difficult incorporation of BrdU into DNA, a classical cell cycle assay with propidium iodide was proposed. Through flow cytometric analysis of the amount of DNA labeled with propidium iodide, it was observed that cells that received treatment at a concentration of 20 µM for 48 hours showed a statistically significant increase in the proportion of cells arrested in G0 phase/G1 and a reduction in the number of cells in S phase (Figure 49).

Therefore, the loss of cell viability produced after treatment with pasireotide would be contributing to a lower proliferation in non-tumor cell lines.

### Example 55. Evaluation of the effect of pasireotide on breast cancer cell lines

In the treatment of mice with mammary tumor changes in tumor evolution were observed; In general, a lower aggressiveness of the disease was observed. Therefore, the effect of pasireotide on cell viability in breast cancer lines was studied. Two cell lines of the same intrinsic tumor subtype as those developed by the two mouse models were used: the tumor cell lines HCC1937 (derived from a triple-negative tumor, deficient in BRCA1) and AU565 (HER2/NEU positive and luminal B).

First, the expression of somatostatin receptors in these lines was studied by QPCR. Expression of the four receptors that have affinity for pasireotide was observed, with SSTR2 showing a higher level of expression in both tumor lines (Figure 50).

### Example 56. Evaluation of the effect of pasireotide on cell viability

As in previous studies, the effect of different concentrations of pasireotide (5 µM, 10 µM and 20 µM) and exposure times (48 and 72 hours) on the viability of tumor cells was evaluated using MTT assays. In the viability assay in the HCC1937 cell line, a 53% reduction was observed, at a concentration of 20 µM of pasireotide, after 48 hours of exposure (Figure 51A); therefore, this concentration was used in the following studies with this cell line.

In the AU565 cell line, a decrease in viability of 52.3% was observed after 48 hours of treatment with pasireotide, at a concentration of 20 µM (Figure 51B).

Differences in cell viability were confirmed under phase-contrast microscopy. Both cell lines, in the presence of treatment, showed less confluency in the plate, as well as more detached cells (Figure 52).

### Example 57. Evaluation of the effect of pasireotide on cell proliferation

Once the optimum concentration was obtained at which cell viability was reduced by half, a proliferation study was carried out by incorporating BrdU into cellular DNA in the HCC1937 line. Subsequently, the extent to which pasireotide could affect the proliferation of tumor cell lines and, thereby, decrease viability was evaluated. The same dose of 20 µM was used for 48 hours of exposure, with which the greatest reduction was previously observed.

In HCC1937 cells treated with pasireotide, a statistically significant decrease in proliferation (proportion of cells in S phase) was observed, together with a reduction in the number of cells in G2/M phases and an increase in cells in G0/G1 phase (Figure 53).

The low proliferation of the AU565 line made it difficult to adjust the exposure time to BrdU, so, initially, it was preferred to use classical cell cycle assays with propidium iodide. Thus, with flow cytometric analysis of the amount of DNA labeled with propidium iodide, it was observed that cells treated with pasireotide at a concentration of 20 µM for 48 hours showed a statistically significant increase in the proportion of cells arrested. in G0/G1 phase, a reduction in the number of cells in S phase and no differences were found in G2/M phase (Figure 54).

## Claims

1. Pasireotide or a pharmaceutically acceptable salt or solvate thereof, for use in prevention or delay of appearance or development of post-pregnancy breast cancer in a female mammal.

2. Pasireotide or a pharmaceutically acceptable salt or solvate thereof, for use according to claim 1, wherein said pasireotide or said salt or solvate is selected from the group consisting of pasireotide, pasireotide acetate, pasireotide L-aspartate, pasireotide D-aspartate, pasireotide diaspartate, pasireotide aspartate-trifluoroacetate, pasireotide aspartate-trifluoroacetate and pasireotide pamoate.

3. Pasireotide or a pharmaceutically acceptable salt or solvate thereof, for use according to any one of claims 1 and 2, wherein said breast cancer is metastatic.

4. Pasireotide or a pharmaceutically acceptable salt or solvate thereof, for use according to any one of claims 1 to 3, wherein said breast cancer exhibits cellular pleomorphism, more than 16 mitoses and no type of conserved structure.

5. Pasireotide or a pharmaceutically acceptable salt or solvate thereof, for use according to any one of claims 1 to 4, wherein said female mammal is a woman.

6. Pasireotide or a pharmaceutically acceptable salt or solvate thereof, for use according to any one of claims 1 to 5, wherein said female mammal has a mutation in the *Brca1* gene and/or the *P53* gene and/or the *ErbB2* gene.

7. Pasireotide or a pharmaceutically acceptable salt or solvate thereof, for use according to any one of claims 1 to 6, wherein said breast cancer is a ductal epithelial breast cancer.

8. Pasireotide or a pharmaceutically acceptable salt or solvate thereof, for use according to any one of claims 1 to 7, wherein said female animal expresses a somatostatin receptor in epithelial breast cells.

9. Pasireotide or a pharmaceutically acceptable salt or solvate thereof, for use according to claim 8, wherein said somatostatin receptor is encoded by a gene selected from the group consisting of: *Sstr1*, *Sstr2, Sstr3* and *Sstr5.*

10. Pasireotide or a pharmaceutically acceptable salt or solvate thereof, for use to any one of claims 1 to 9, wherein said female mammal has weaned their offspring.

11. Pasireotide or a pharmaceutically acceptable salt or solvate thereof, for use according to any one of claims 1 to 10, wherein said pasireotide or said salt or solvate is administered to said female mammal during and/or after post-lactational involution.

12. Pasireotide or a pharmaceutically acceptable salt or solvate thereof, for use according to any one of claims 1 to 11, wherein said woman is ≥ 30 years old when having given birth.

13. Pasireotide or a pharmaceutically acceptable salt or solvate thereof, for use according to claim 12, wherein said woman is ≥ 35 years old when having given birth.

14. Pasireotide or a pharmaceutically acceptable salt or solvate thereof, for use according to any one of claims 1 to 13, wherein said woman was pregnant for more than 34 weeks.
